# EUROPEAN PATENT APPLICATION

(11) **EP 4 722 227 A1**
(43) Date of publication of application: **08.04.2026**
(21) Application number: 24810971.2
(22) Date of filing: 14.05.2024
(51) Int. Cl.: C07K 2/00, A61K 39/00, A61K 39/385, A61P 35/00, C12N 5/078, C12N 5/09, C12N 9/26, C12P 21/08

(54) **COMPLEX, PHARMACEUTICAL COMPOSITION, VACCINE, AND APPLICATIONS THEREOF**

(30) Priority: 25.05.2023 JP 2023086026
(71) Applicant: ENU Pharma, Inc., Sapporo-shi Hokkaido 001-0021 (JP)
(72) Inventor: NISHIMURA Shin-Ichiro, Sapporo-shi, Hokkaido 060-0808 (JP); WAKUI Hajime, Sapporo-shi, Hokkaido 060-0808 (JP); YOKOI Yasuhiro, Sapporo-shi, Hokkaido 060-0808 (JP)
(74) Representative: Godemeyer Blum Lenze Patentanwälte Partnerschaft mbB - werkpatent
(86) International application number: PCT/JP2024/017786
(87) International publication number: WO 2024/241964

(57) **Abstract**

It is an object of the present invention to provide a conjugate for obtaining a monoclonal antibody that simultaneously and specifically recognizes both glycans and peptides that constitute a glycopeptide that is a dynamic epitope. The present invention relates to a conjugate represented by the following formula (1): 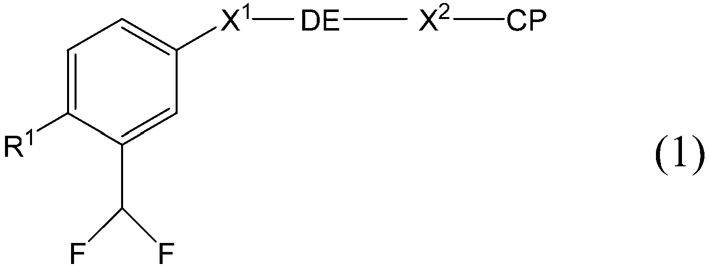 wherein R¹ represents a neuraminic acid group or a neuraminic acid derivative group, DE represents an antigenic site selected from a glycoprotein and a glycopeptide, CP represents a carrier site, and X¹ and X² each independently represent a single bond or a linking group.

## Description

### Technical Field

The present invention relates to a conjugate, a pharmaceutical composition, a vaccine, a method for obtaining antigen presenting cells, a method for generating an antibody, and a method for treating a tumor and/or a cancer. The present invention relates to providing a conjugate that is useful for generating antibodies against epitopes, for which it has been difficult to generate antibodies so far.

### Background Art

The greatest and most serious challenge in developing therapeutic antibodies is "depletion of antigens" specific for diseases. Since monoclonal antibodies have been vigorously developing over the world, antibodies that can be obtained by common methods have been nearly exhausted under the current circumstances. Therefore, recent priorities have shifted to the development of various modified antibody-related pharmaceutical products, such as bispecific antibodies, antibody-drug conjugates, and further, chimeric antigen T cells, which are developed for the purpose of improving efficacy and safety. Research into disease-specific, yet unclarified antigens (target molecules), regarding which the development of novel antibody drug development is expected, has hardly progressed under the current circumstances.

In 2009, the present inventors have demonstrated for the first time "the existence of antibodies that simultaneously recognize and bind to both regions of a glycopeptide consisting of glycans and peptides as a single molecule" at the molecular level (Non-Patent Document 1: Nishimura, S.-I. et al., An essential epitope of anti-MUC1 monoclonal antibody KL-6 revealed by focused glycopeptide library. J. Am. Chem. Soc. 2009, 131, 17102-17109). Furthermore, the present inventors have revealed that "the binding specificity and affinity between such antibodies and cancer-specific antigenic determinants (epitopes) are strongly influenced by post-translational glycosylation in this antigen peptide region (Non-Patent Document 2: Nishimura, S.-I. et al., Convergent solid-phase synthesis of macromolecular MUC1 models truly mimicking serum glycoprotein biomarkers of interstitial lung diseases. J. Am. Chem. Soc. 2016, 138, 8392-8395).

These findings have led to the proposal of a novel principle, "dynamic epitope theory" that can be generalized as a novel molecular mechanism for the formation of protein antigen structures. Since the "dynamic epitopes," which are discovered in various diseases, represent a treasure trove of antigens that can serve as novel target molecules, the dynamic epitopes are expected to become a key tool in solving the problem of "antigen depletion."

However, it has become clear that it is difficult to obtain antibodies that specifically bind to such promising target molecules, "dynamic epitopes," with high affinity, and that it requires trial and error immunization methods and multi-stage antibody screening (e.g., Non-Patent Document 3: Nishimura, S.-I. et al., A straightforward approach to antibodies recognizing cancer specific glycopeptidic neoepitopes. Chem. Sci., 2020, 11, 4999-5006). It is considered because glycopeptides (dynamic epitopes) that should be recognized by B cells as antigens (B cell-epitopes) through immunization have the following problems:
- after the glycopeptides (dynamic epitopes) have been incorporated into dendritic cells as antigen presenting cells, or macrophages, the glycan region is easily decomposed and removed by a group of various glycosidases, during a lysosomal processing until the glycopeptides (dynamic epitopes) bind to MHC I/II (Major Histocompatibility Complex class I/II, mainly MHC II); and further,
- originally, the glycopeptides (dynamic epitopes) themselves, which are B cell-epitopes, have low affinity with the antigen peptide (T cell-epitope)-binding region of MHC molecules (e.g., Non-Patent Document 4: Germain, R. N. MHC-dependent antigen processing and peptide presentation: Providing ligands for T lymphocyte activation. Cell 1994, 75, 287-299; Non-Patent Document 5:Blum, J. S., et al., Pathways of antigen processing. Annu. Rev. Immunol. 2013, 31, 443-473). Therefore, there has been an urgent need to establish a novel methodology for obtaining monoclonal antibodies that simultaneously and specifically recognize both the glycans and peptides that constitute a glycopeptide that is a dynamic epitope.

### Prior Art Documents

### Patent Documents

Patent Document 1: WO2017/131242

### Non-Patent Documents

Non-Patent Document 1: Nishimura, S.-I. et al., J. Am. Chem. Soc. 2009, 131, 17102-17109
Non-Patent Document 2: Nishimura, S.-I. et al., J. Am. Chem. Soc. 2016, 138, 8392-8395
Non-Patent Document 3: Nishimura, S.-I. et al., Chem. Sci., 2020, 11, 4999-5006
Non-Patent Document 4: Germain, R. N. Cell 1994, 75, 287-299
Non-Patent Document 5: Blum, J. S., et al., Annu. Rev. Immunol. 2013, 31, 443-473

### Summary of Invention

### Objects to be Solved by the Invention

It is an object of the present invention to provide a conjugate for obtaining a monoclonal antibody that simultaneously and specifically recognizes both regions of a glycopeptide consisting of glycans and peptides as a single molecule.

### Means for Solving the Objects

Specifically, the present invention provides the following aspects to achieve the aforementioned object:
[1] A conjugate represented by the following formula (1): 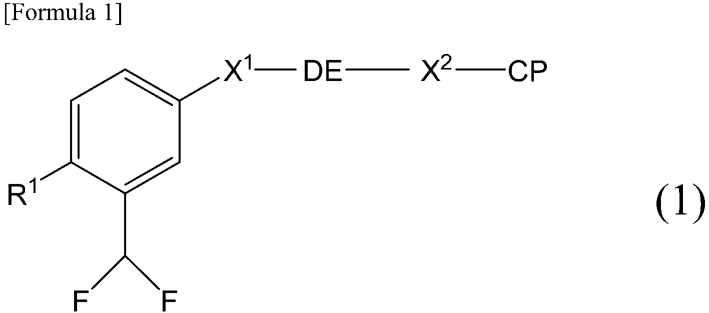 wherein R¹ represents a neuraminic acid group or a neuraminic acid derivative group, DE represents an antigenic site selected from a glycoprotein and a glycopeptide, CP represents a carrier site, and X¹ and X² each independently represent a single bond or a linking group.
[2] The conjugate according to [1], wherein, in the above formula (1), R¹ is a group represented by the following formula (2): 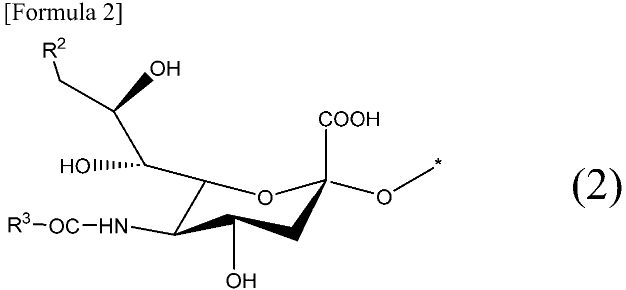 wherein R² represents a group containing at least one type selected from the group consisting of a carbon atom, an oxygen atom and a nitrogen atom, and also containing 10 or less carbon atoms, R³ represents a hydrocarbon group containing 1 to 12 carbon atoms, and * represents a binding site with a benzene ring in the formula (1).
[3] The conjugate according to [2], wherein, in the above formula (2), R² represents a hydroxy group, an alkyl group containing 1 to 6 carbon atoms, or an alkylamide group containing 1 to 6 carbon atoms.
[4] The conjugate according to [2] or [3], wherein, in the above formula (2), R³ represents an alkyl group containing 1 to 3 carbon atoms.
[5] The conjugate according to any one of [1] to [4], wherein the carrier site is at least one type selected from the group consisting of lipid nanoparticles, metal nanoparticles, polysaccharides, and carrier proteins.
[6] The conjugate according to any one of [1] to [5], which is used to bind to cells having Neuraminidase 1 (NEU1) or Neuraminidase 3 (NEU3).
[7] The conjugate according to any one of [1] to [6], which is for use in antibody generation.
[8] A pharmaceutical composition, comprising the conjugate according to any one of [1] to [7].
[9] The pharmaceutical composition according to [8], which is used in prevention and/or treatment of a tumor and/or a cancer.
[10] A vaccine, comprising the conjugate according to any one of [1] to [7].
[11] A method for obtaining antigen presenting cells, which present the antigenic site and the carrier site in the above formula (1) on the cell surface thereof,
   the method comprising
   allowing the conjugate according to any one of [1] to [7] to come into contact with cells having Neuraminidase 1 (NEU1) or Neuraminidase 3 (NEU3).
[12] A method for generating an antibody, comprising generating an antibody using the antigen presenting cells according to [11].
[13] A method for treating a tumor and/or a cancer, comprising:
   administering the conjugate according to any one of [1] to [7], and
   administering an antibody binding to the antigenic site in the above formula (1).
[14] Use of the conjugate according to any one of [1] to [7] for production of a preventive and/or therapeutic agent for a tumor and/or a cancer.
[15] A conjugate represented by the following formula (11): 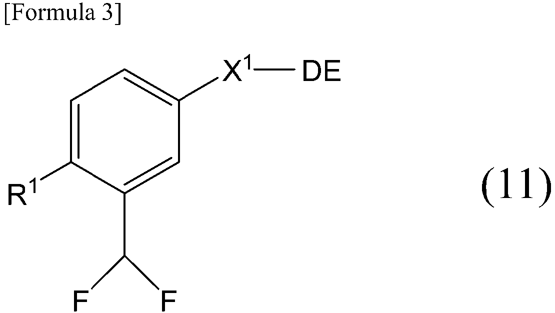 wherein R¹ represents a neuraminic acid group or a neuraminic acid derivative group, DE represents an antigenic site selected from a glycoprotein and a glycopeptide, and X¹ and X² each independently represent a single bond or a linking group.
[16] The conjugate according to [15], wherein, in the above formula (11), R¹ is a group represented by the following formula (2): 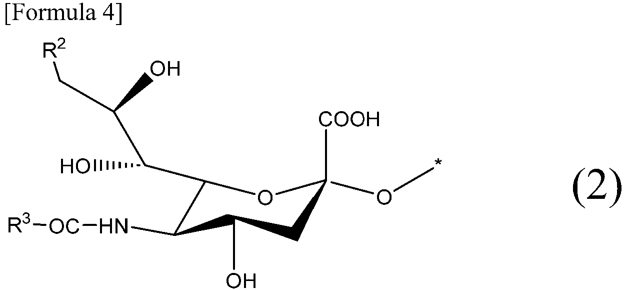 wherein R² represents a group containing at least one type selected from the group consisting of a carbon atom, an oxygen atom and a nitrogen atom, and also containing 10 or less carbon atoms, R³ represents a hydrocarbon group containing 1 to 12 carbon atoms, and * represents a binding site with a benzene ring in the formula (1).
[17] The conjugate according to [16], wherein, in the above formula (2), R² represents a hydroxy group, an alkyl group containing 1 to 6 carbon atoms, or an alkylamide group containing 1 to 6 carbon atoms.
[18] The conjugate according to [16] or [17], wherein, in the above formula (2), R³ represents an alkyl group containing 1 to 3 carbon atoms.
[19] A pharmaceutical composition, comprising the conjugate according to any one of [15] to [18].
[20] A method for obtaining antigen presenting cells, which present the antigenic site and the carrier site in the above formula (11) on the cell surface thereof,
   the method comprising
   allowing the conjugate according to any one of [15] to [18] to come into contact with cells having Neuraminidase 1 (NEU1) or Neuraminidase 3 (NEU3).

[101]: A compound having the following formula: 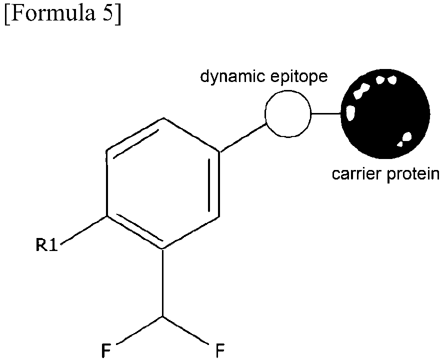 wherein
   R1 represents neuraminic acid,
   the dynamic epitope is selected from the group consisting of a protein or a peptide, a glycan, and a conjugate of a protein or a peptide and a glycan, and
   the carrier protein is a protein that exhibits an immunogenic action when it is combined with a dynamic epitope.
[102]: The compound according to [101], for generation of an antibody against a dynamic epitope.
[103]: The compound according to [101] or [102], which is capable of irreversibly binding with Neuraminidase 1 (NEU1) or Neuraminidase 3 (NEU3) to form a conjugate comprising NEU1 or NEU3, a dynamic epitope, and a carrier protein.
[104]: The compound according to [103], wherein NEU1 or NEU3 exists as a membrane protein in the cell membrane.
[105]: The compound according to [104], wherein the dynamic epitope and carrier protein portions of the compound that form a conjugate with NEU1 or NEU3 in the cell membrane are presented outside of the cells.
[106]: A method for forming a conjugate comprising NEU1 or NEU3, a dynamic epitope, and a carrier protein comprising:
   irreversibly binding a compound having the following formula with Neuraminidase 1 (NEU1) or Neuraminidase 3; 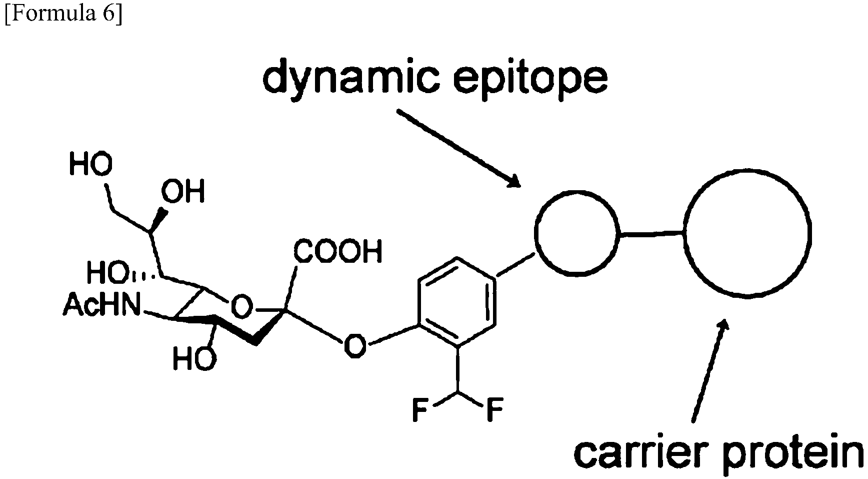
   [wherein
   the dynamic epitope is selected from the group consisting of a protein or a peptide, a glycan, and a conjugate of a protein or a peptide and a glycan, and
   the carrier protein is a protein that exhibits an immunogenic action when it is combined with a dynamic epitope].
[107]: The method according to [106], which causes a nucleophilic reaction between the F of the difluoromethylaryl group of the compound and the nucleophilic side chains of glutamic acid or aspartic acid residues, etc., in or near the enzyme active center of NEU1 or NEU3.
[108]: The method according to [106] or [107], wherein NEU1 or NEU3 exists as a membrane protein in the cell membrane.
[109]: The method according to [108], wherein the dynamic epitope and carrier protein portions of the above-described compound are presented outside of the cells, when the above-described compound forms a conjugate with NEU1 or NEU3 that exists as a membrane protein in the cell membrane.
[110]: A method for generating an antibody against a dynamic epitope, comprising:
   irreversibly binding a compound having the following formula with Neuraminidase 1 (NEU1) or Neuraminidase 3 (NEU3), to form a conjugate comprising NEU1 or NEU3, a dynamic epitope, and a carrier protein; and
   presenting the dynamic epitope and the carrier protein in the conjugate to target cells. 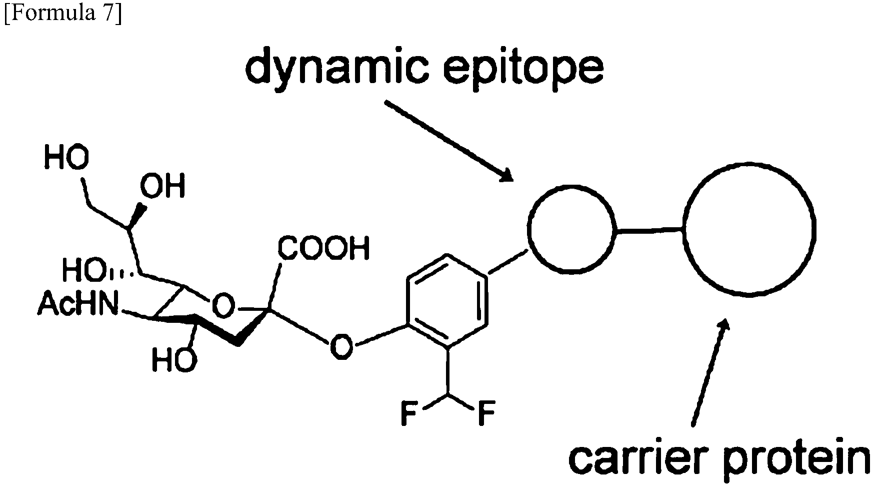
   [wherein
   the dynamic epitope is selected from the group consisting of a protein or a peptide, a glycan, and a conjugate of a protein or a peptide and a glycan, and
   the carrier protein is a protein that exhibits an immunogenic action when it is combined with a dynamic epitope].

### Advantageous Effects of Invention

According to the present invention, there can be provided a conjugate for obtaining a monoclonal antibody that simultaneously and specifically recognizes both regions of a glycopeptide consisting of glycans and peptides as a single molecule.

### Brief Description of Drawings

[Figure 1] Figure 1 is a view showing that the enzyme activity of a scaffold protein present as a membrane protein in a cell membrane is inhibited by a specific reaction mechanism using the compound of the present invention, and the compound of the present invention is allowed to irreversibly bind to the scaffold protein, so that a conjugate comprising the scaffold protein and a dynamic epitope (and a carrier protein) binding to the compound of the present invention can be formed, while using NEU1 as an example of the scaffold protein.
[Figure 2] Figure 2 is a view showing that the monoclonal antibody clones produced and screened by the method of the present invention bind only to the glycopeptide and do not exhibit affinity for the main chain peptide.

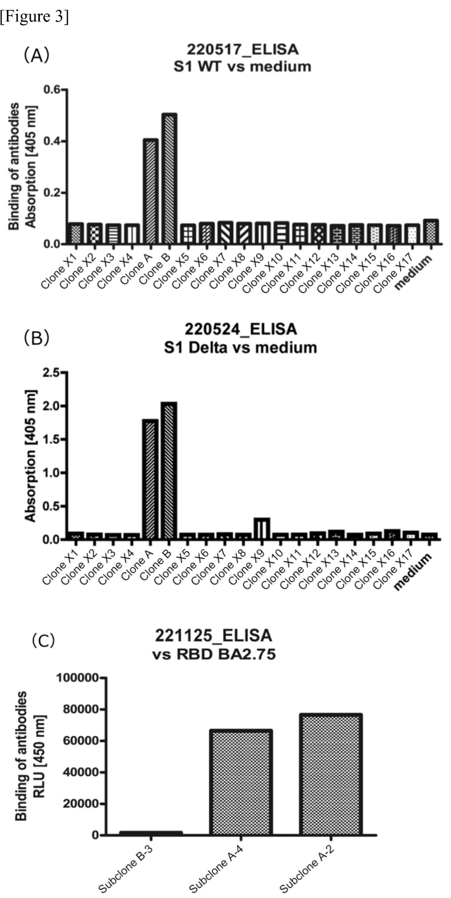

[Figure 3] Figure 3 is a view showing that the monoclonal antibody clones produced by the method of the present invention bind strongly to the spike glycoprotein (S1 WT) of the SARS-CoV-2 wild strain and the spike glycoprotein (S1 Delta) of the Delta strain (Figure 3(A) and Figure 3(B)), and that some subclones also bind strongly to the RBD of the recombinant spike glycoprotein of the Omicron strain (BA.2.75) (Figure 3(C)). Figure 3(C) shows that the monoclonal antibody clones produced by the method of the present invention bind strongly to the spike glycoprotein (S1 WT) of the SARS-CoV-2 wild strain and the spike glycoprotein (S1 Delta) of the Delta strain (Figure 3(A) and Figure 3(B)). 3(B)), and that some subclones also strongly bind to the RBD of the recombinant spike glycoprotein of the Omicron strain (BA.2.75).
[Figure 4] Figure 4 is a view showing that the monoclonal antibody clones produced by the method of the present invention strongly bind to the spike glycoprotein (S1 WT) of the SARS-CoV-2 wild strain at the order of nM.
[Figure 5] Figure 5 is a view showing representative examples of the screening results using antibodies obtained by the conventional method and the reactivity of these antibodies with the spike glycoprotein (S1) derived from the SARS-CoV-2 wild strain.
[Figure 6] Figure 6 is a view showing that the compound of the present invention was utilized to immobilize a dynamic epitope to NEU1 present on the surface of cancer cells, and as a result that antibodies specific to the dynamic epitope can be induced to cancer cells, and can be incorporated into the cancer cells.
[Figure 7] Figure 7 is a view showing a scheme for inducing anti-MUC1 antibodies to cancer cells by allowing a dynamic epitope (target molecule) that is the binding target of anti-MUC1 antibodies to bind to NEU1 sialidase present on the cell surface of cancer cells.
[Figure 8] Figure 8 is a view showing that the monoclonal antibody clones produced and screened by the method of the present invention all bind only to the glycopeptide and do not exhibit affinity for the main chain peptide.
[Figure 9] Figure 9 is a view showing that the monoclonal antibody clones produced by the method of the present invention all bind only to the glycopeptide and do not exhibit affinity for the main chain peptide.
[Figure 10] Figure 10 is a view showing that a monoclonal antibody against fibronectin glycoprotein strongly binds to glycopeptides with Sialyl-Tn binding to two sites, Thr2155 and Thr2156, but does not bind to glycopeptides with other glycosylations (Tn or T) or glycopeptides with Sialyl-Tn binding to three sites, Thr2152, Thr2155, and Thr2156.
[Figure 11] Figure 11 is a view showing that a monoclonal antibody against fibronectin glycoprotein strongly binds to glycopeptides with Sialyl-Tn binding to two sites, Thr2155 and Thr2156, but does not bind to glycopeptides with other glycosylations (Tn or T) or glycopeptides with Sialyl-Tn linked at three sites, Thr2152, Thr2155, and Thr2156.

### Embodiments of Carrying out the Invention

The present invention will be described in detail below. The explanation given below may be described based on some typical embodiments or specific examples. However, the present invention should not be limited to such embodiments. It is to be noted that, in the present description, the "dynamic epitope" refers to a glycopeptide (glycoprotein) formed by linking a glycan to a peptide (protein),which appears as a result of post-translational glycosylation, etc. (differences in the modification site and the glycan structure).

In the present description, as represented by Formula (1) or Formula (11) below, a compound, in which a difluoromethylaryl skeleton is linked to a "dynamic epitope," is referred to as a "conjugate." It is to be noted that, in the present description, the "conjugate" is referred to as a compound at times, and thus, the conjugate encompasses compounds. Also, in the present description, glycopeptide and glycoprotein are collectively referred to simply as a "glycopeptide," at times.

The inventors of the present invention have conducted intensive studies aimed at generating antibodies against glycopeptides (glycoproteins), the generation of which has previously been difficult. As a result, the present inventors have demonstrated that there can be provided a conjugate that is useful for establishing a novel methodology for obtaining monoclonal antibodies that simultaneously and specifically recognize both the glycans and peptides that constitute a glycopeptide that is a dynamic epitope. Moreover, the inventors of the present invention have demonstrated that there can be provided a method of efficiently presenting glycopeptides (glycoproteins) as antigens based on a novel mechanism that is not dependent on the binding with MHC molecules, by utilizing the aforementioned conjugate. In such a novel mechanism that is not dependent on the binding with MHC molecules, since the glycan region of the glycopeptide (glycoprotein) is effectively presented and recognized by B cells without being decomposed (i.e., antigen-specific B cells are induced), the efficiency of generating antibodies that specifically bind to the "dynamic epitopes" as antigenic structures of interest consisting of glycans and peptides can be dramatically improved, and it also becomes possible to efficiently incorporate cancer therapeutic antibodies that bind to the dynamic epitopes into cancer cells.

### (Conjugate)

A first embodiment of the present invention relates to a conjugate represented by the following formula (1):

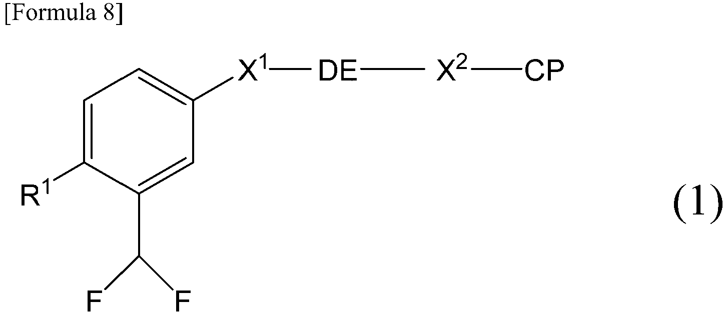

In the above formula (1), R¹ represents a neuraminic acid group or a neuraminic acid derivative group, DE represents an antigenic site selected from a glycoprotein and a glycopeptide, CP represents a carrier site, and X¹ and X² each independently represent a single bond or a linking group. It is to be noted that, in the present description, R¹ is a substituent and is either a neuraminic acid group or a neuraminic acid derivative group. However, R¹ is simply referred to as a neuraminic acid or a neuraminic acid derivative in some cases. In such cases, the neuraminic acid can be interpreted as a group derived from neuraminic acid (i.e., a neuraminic acid group), and the neuraminic acid derivative can be interpreted as a group derived from a neuraminic acid derivative (i.e., a neuraminic acid derivative group).

The conjugate represented by the formula (1) has a neuraminic acid group or a neuraminic acid derivative group as R¹ linked to a difluoromethylaryl skeleton, an antigenic site selected from a glycoprotein and a glycopeptide as DE linked to the difluoromethylaryl skeleton, and a carrier site (CP) linked to the DE.

In the formula (1), DE represents an antigenic site selected from a glycoprotein and a glycopeptide. The glycoprotein is a protein having a glycan, and the glycopeptide is a peptide having a glycan. In the present description, a substance formed by binding 50 or more amino acids together is called a protein, and a substance formed by binding less than 50 amino acids together is called a peptide. In addition, in the present description, the "glycan" refers to a compound that can be formed by linking one or more sugar units (monosaccharides and/or their derivatives). When two or more sugar units are linked together, the spaces between individual sugar units are allowed to bind to one another by dehydration condensation via a glycosidic bond. It is to be noted that the sugars that constitute the glycan may be monosaccharides, or that the glycan may consist of 1, 2, 3, 4, 5, 6, 7, or 8 sugars. In the present invention, the glycan possessed by the glycoprotein or the glycopeptide may be a glycan having four O-glycan structures (Tn, Sialyl-Tn, T, and Sialyl-T) that have been known to be observed in cancer cells.

In the formula (1), the number of glycans possessed by the DE is not particularly limited, as long as the DE has one or more glycans. The upper limit of the number of glycans possessed by the DE is preferably, for example, 20 or less, more preferably 15 or less, and further preferably 10 or less.

In the formula (1), the DE is preferably a glycopeptide. The glycopeptide is preferably composed of, for example, 5 to 40 amino acid residues, and is more preferably composed of 10 to 30 amino acid residues. In this case, the glycopeptide preferably has 1 to 5 glycans.

The glycoprotein or the glycopeptide represented by the DE is not particularly limited, as long as it can serve as an antigenic site. Examples of the glycoprotein or the glycopeptide represented by the DE may include various MUC1 glycopeptides disclosed in International Publication No. WO2010/050528 and International Publication No. WO 2011/135869, and glycopeptides produced by the method of synthesizing glycopeptides disclosed in JP Patent Publication (Kokai) No. 2012-167018 A.

In the formula (1), CP represents a carrier site. The carrier site enhances antigenicity when antibodies are produced using the conjugate of the present invention, and the carrier site is a site that exhibits immunogenicity together with the antigenic site DE. It is to be noted that since the carrier site plays an role of increasing the overall molecular weight together with the aforementioned antigenic site (DE) and thereby allowing the immunogenicity of the antigenic site (DE) to exhibit, it is preferable that the carrier site (CP) itself does not have antigenicity.

The carrier site preferably has a predetermined particle diameter to enhance the antigenicity of the antigenic site (DE). The average particle diameter of the carrier site is preferably 1 nm or more, more preferably 3 nm or more, and further preferably 5 nm or more. On the other hand, the average particle diameter of the carrier site is preferably 500 nm or less, more preferably 300 nm or less, and further preferably 100 nm or less.

The carrier site is not particularly limited, as long as it plays a role of allowing the immunogenicity of the antigenic site (DE) to exhibit by increasing the molecular weight of the antigenic site (DE). For example, the carrier site is preferably at least one type selected from the group consisting of lipid nanoparticles, metal nanoparticles, polysaccharides, and carrier proteins. The lipid nanoparticles are nanoparticles comprising lipids as main components, and examples thereof may include nanoparticles composed of lipid membranes, such as liposomes and exosomes. The metal nanoparticles may include gold nanoparticles, silver nanoparticles, copper nanoparticles, platinum nanoparticles, and quantum dots. The polysaccharides may include chitosan, chitin, dextrin, hyaluronic acid, and pectin. The average molecular weight of the polysaccharide is preferably 1,000 or more, and more preferably 3,000 or more. The average molecular weight of the polysaccharide is preferably 5,000,000 or less, and more preferably 1,000,000 or less.

Among these, the carrier site is preferably a carrier protein. Various types of proteins can be used as such carrier protein. Examples of the carrier protein may include: high-molecular-weight substances such as keyhole limpet hemocyanin (KLH), concholepas concholepas hemocyanin (CCH), bovine serum albumin (BSA), ovalbumin (OVA), granulocyte-macrophage colony-stimulating factor (GM-CSF), bovine thyroglobulin (BTG), and chicken gamma globulin (CGG); and synthetic polypeptides. The average molecular weight of the carrier protein is preferably 5 kDa or more, more preferably 10 kDa or more, and further preferably 15 kDa or more. On the other hand, the average molecular weight of the carrier protein is preferably 2000 kDa or less, more preferably 1000 kDa or less, and furhter preferably 500 kDa or less.

In the formula (1), the difluoromethylaryl skeleton and the DE may be directly linked to each other. Otherwise, a linking group may also exist between the difluoromethylaryl skeleton and the DE. That is, in the formula (1), X¹ is a single bond or a linking group. If X¹ is a linking group, the linking group is preferably a linking group containing at least one type selected from the group consisting of a carbon atom, an oxygen atom, and a nitrogen atom. More specifically, the linking group is preferably a divalent hydrocarbon group, or a group formed by combining a divalent hydrocarbon group with one or more of -O-, -NR-, -C(=O)-, and -N=.

In the formula (1), the DE and carrier site (CP) may be directly linked to each other. Otherwise, a linking group may also exist between the DE and carrier site (CP). That is, in the formula (1), X² is a single bond or a linking group. If X² is a linking group, the linking group preferably a linking group containing at least one type selected from the group consisting of a carbon atom, an oxygen atom, and a nitrogen atom. More specifically, the linking group is preferably a divalent hydrocarbon group, or a group formed by combining a divalent hydrocarbon group with one or more of -O-, -NR-, - C(=O)-, and -N=.

In the formula (1), when X¹ and X² are each independently a linking group, the linking group preferably has an amide bond. Moreover, the molecular weight of the linking group is preferably 500 or less, more preferably 400 or less, and further preferably 300 or less.

In the formula (1), the difluoromethylaryl skeleton, to which R¹ is linked, is a structure derived from a NEU1 sialidase inhibitor or a NEU3 sialidase inhibitor, and R¹ is a neuraminic acid group or a neuraminic acid derivative group. Among these, R¹ is preferably a neuraminic acid derivative group. It is to be noted that, in the present description, the neuraminic acid derivative group may be, for example, a group represented by the following formula (2). That is, in the formula (1), R¹ is preferably a group represented by the following formula (2):

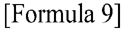

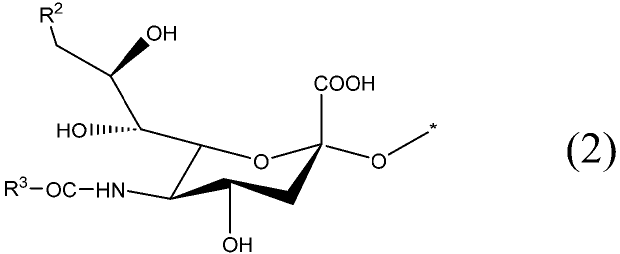

In the above formula (2), R² and R³ are not particularly limited, as long as R² and R³ are each independently a substituent that does not inhibit the binding with Neuraminidase 1 (NEU1) or Neuraminidase 3 (NEU3). In the present embodiment, R² and R³ can be selected appropriately, taking into consideration the degree of binding affinity with Neuraminidase 1 (NEU1) or Neuraminidase 3 (NEU3). In the present embodiment, it is preferable that R² is a group containing at least one type selected from the group consisting of a carbon atom, an oxygen atom and a nitrogen atom, and also containing 10 or less carbon atoms, that R³ is a hydrocarbon group containing 1 to 12 carbon atoms, and that * is preferably a binding site with a benzene ring in the formula (1).

In the formula (2), R² is preferably a group containing at least one type selected from the group consisting of a carbon atom, an oxygen atom and a nitrogen atom, and also containing 10 or less carbon atoms, and is more preferably a group containing at least one type selected from the group consisting of a carbon atom and an oxygen atom, and also containing 10 or less carbon atoms. Examples of R² may include a hydroxy group, an alkyl group containing 1 to 10 carbon atoms, an alkoxy group containing 1 to 10 carbon atoms, and an alkylamide group containing 1 to 10 carbon atoms. Among these, R² is preferably a hydroxy group, an alkyl group containing 1 to 6 carbon atoms, an alkoxy group containing 1 to 6 carbon atoms, or an alkylamide group containing 1 to 6 carbon atoms; more preferably a hydroxy group, an alkyl group containing 1 to 6 carbon atoms, or an alkylamide group containing 1 to 6 carbon atoms; and particularly preferably a hydroxy group.

In the formula (2), R³ is preferably a hydrocarbon group containing 1 to 12 carbon atoms, is more preferably a hydrocarbon group containing 1 to 10 carbon atoms, and is further preferably a hydrocarbon group containing 1 to 6 carbon atoms. Examples of the hydrocarbon group may include aliphatic hydrocarbon groups (e.g., an alkyl group, an alkenyl group, an alkynyl group, and a cycloalkyl group) and aromatic hydrocarbon groups (e.g., a phenyl group and a naphthyl group). Among these, R³ is preferably an alkyl group containing 1 to 6 carbon atoms or a phenyl group; more preferably an alkyl group containing 1 to 3 carbon atoms; and particularly preferably a methyl group.

Conventionally, when a glycoprotein or a glycopeptide intends to be presented on the cell surface as an antigen, the glycoprotein or the glycopeptide is incorporated into antigen presenting cells such as dendritic cells or macrophages, and then, during lysosomal processing before binding with MHC I/II (Major Histocompatibility Complex class I/II, mainly MHC II), the glycan region is easily decomposed and eliminated by a group of various glycosidases. As a result, it has been impossible to obtain monoclonal antibodies that simultaneously recognize both the glycans and peptides that constitute the glycopeptide, which is a dynamic epitope. However, in the present invention, by using a conjugate as shown in the above structural formula, it has been succeeded in presenting the antigen on the cell surface according to an MHC-independent method that is not dependent on the binding with MHC molecules. Thereby, since the antigen is presented without decomposing and eliminating glycans, a monoclonal antibody simultaneously recognizing both the glycan and the peptide, which constitute the glycopeptide that is a dynamic epitope, can be obtained. For example, in the case of cancer, etc., it has been known that proteins on the cell surface proteins are modified with glycans, so that they are avoided from the immune system of a living body. It is considered that effective generation of antibodies against dynamic epitopes could provide novel therapeutic tools for a variety of diseases. Thus, by using the compound (conjugate) of the present invention, highly specific antibodies against dynamic epitopes can be generated, and the resulting antibodies can be used in the diagnosis and treatment of diseases such as cancers.

In one embodiment, the conjugate of the present invention may be a compound having the following formula:

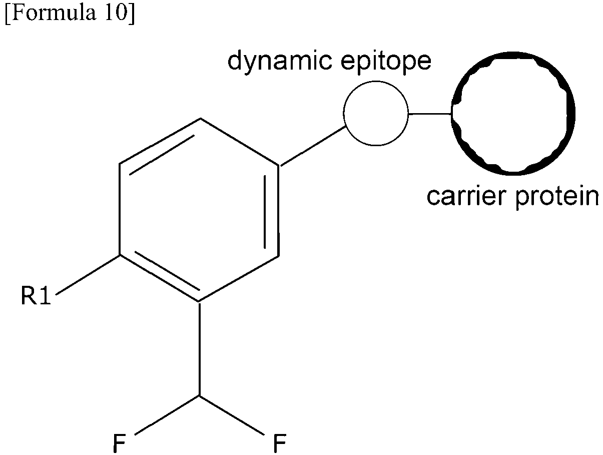

In the above formula, R1 is neuraminic acid,
the dynamic epitope is selected from the group consisting of a protein or a peptide, a glycan, and a conjugate of a protein or a peptide and a glycan, and
the carrier protein is a protein that exhibits an immunogenic action when it is combined with a dynamic epitope.

This compound has a structure, in which neuraminic acid as R1 of the difluoromethylaryl skeleton binds to a molecule in which a dynamic epitope and a carrier protein are bound in series, apart from the R1.

The "dynamic epitope" contained in this compound refers to an epitope structure that is generated by post-translational modification of a protein (in particular, glycosylation) and is not dependent on only the peptide structure. In general, since such a dynamic epitope often has poor antigenicity, it has been known that it is difficult to generate an antibody against this dynamic epitope. The dynamic epitope may be an epitope portion that is not dependent on the primary structure of a protein having a structure in which the glycan binds to the protein, such as, for example, a glycan, or a conjugate of a protein or a peptide and a glycan, etc. In the present invention, however, the dynamic epitope is preferably a glycoprotein or glycopeptide.

Although these dynamic epitopes have a significantly low mutation frequency, previous methods for generating antibodies have generally been unable to generate suitable antibodies. However, it is considered that according to the method of the present invention, a novel therapeutic target that could be previously untargeted can be applied. For example, in the case of cancer, etc., it has been known that proteins on the cell surface proteins are modified with glycans, so that they are avoided from the immune system of a living body. If antibodies against these epitopes can be effectively generated, it is considered that novel therapeutic tools could be provided for various diseases. For this reason, the compound of the present invention is preferable in that it can be used to generate antibodies against dynamic epitopes.

The "carrier protein" contained in this compound refers to a protein having an action to give immunogenicity when it is combined with a dynamic epitope. Examples of proteins that can be used as such carrier proteins may include those commonly known in the present technical field. As such commonly known carrier proteins,keyhole limpet hemocyanin (KLH), concholepas concholepas hemocyanin (CCH), bovine serum albumin (BSA), ovalbumin (OVA), and granulocyte-macrophage colony-stimulating factor (GM-CSF), and the like can be used.

A second embodiment of the present invention relates to a conjugate represented by the following formula (11):

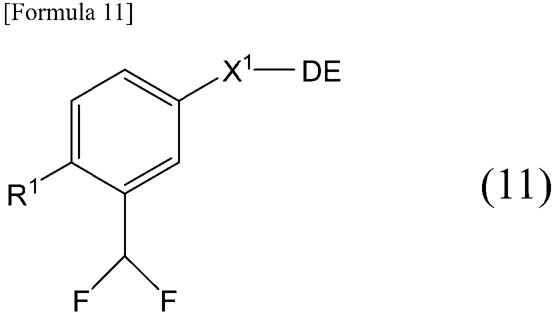

In the above formula (11), R¹ is a neuraminic acid group or neuraminic acid derivative group, DE is an antigenic site selected from a glycoprotein and a glycopeptide, and X¹ and X² each independently represent a single bond or a linking group. R¹ in the formula (11) is the same as R¹ in the above formula (1), and the preferred range is also the same. DE in formula (11) is the same as DE in the above formula (1), and the preferred range is also the same. However, while the N-terminus of DE in the above formula (1) binds to another atom, the N-terminus of DE in the formula (11) does not bind to another atom. Moreover, the N-terminus of DE in the formula (11) may be linked to a capping group or the like.

In the formula (11), R¹ is a neuraminic acid group (a group derived from neuraminic acid) or a neuraminic acid derivative group (a group derived from a neuraminic acid derivative). Of these, R¹ is preferably a neuraminic acid derivative group. The neuraminic acid derivative group may be, for example, the group represented by the above formula (2). That is, in the formula (11), R¹ is preferably the group represented by the above formula (2). The preferred ranges of R² and R³ in the above formula (2) are also the same. In the formula (2), R² is preferably a hydroxy group, an alkyl group containing 1 to 6 carbon atoms, or an alkylamide group containing 1 to 6 carbon atoms, and particularly preferably a hydroxy group. In addition, R³ is preferably an alkyl group containing 1 to 6 carbon atoms, more preferably an alkyl group containing 1 to 3 carbon atoms, and particularly preferably a methyl group.

### (Antigen presentation method)

The conjugate of the present invention is preferably used to bind to cells having Neuraminidase 1 (NEU1) or Neuraminidase 3 (NEU3). As another aspect of the present invention, there can be provided a method for obtaining antigen presenting cells, which present the antigenic site and the carrier site in the following formula (1) on the cell surface thereof, the method comprising allowing the conjugate represented by the formula (1) to come into contact with cells having Neuraminidase 1 (NEU1) or Neuraminidase 3 (NEU3). Moreover, as a further aspect of the present invention, there can be provided an antigen presentation method, comprising allowing the aforementioned conjugate to come into contact with cells having Neuraminidase 1 (NEU1) or Neuraminidase 3 (NEU3).

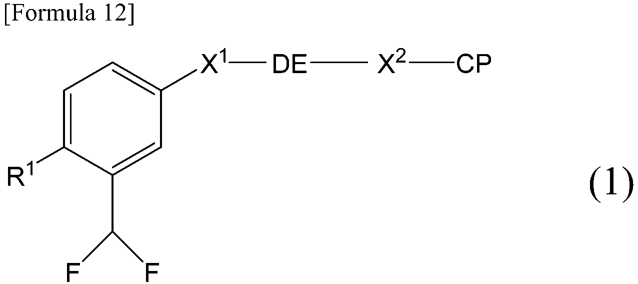

In the above formula (1), R¹ is neuraminic acid or a neuraminic acid derivative, DE is an antigenic site selected from a glycoprotein and a glycopeptide, CP is a carrier site, and X¹ and X² each independently represent a single bond or a linking group.

In the method for obtaining antigen presenting cells, the conjugate represented by the formula (1) is allowed to bind to a scaffold protein (Neuraminidase 1 (NEU1) or Neuraminidase 3 (NEU3)) present on the surface of the cell membrane, so that the dynamic epitope and the carrier site (carrier protein portion) in the conjugate of the present invention can be presented on the surface of the cell membrane to outside of the cell (antigen presentation). As a result, antigen presenting cells can be obtained.

The conjugate of the present invention can be linked to the protein (Neuraminidase 1 (NEU1) or Neuraminidase 3 (NEU3)) serving as a scaffold, via a chemical reaction, an enzymatic reaction, or the like, that occurs between the conjugate of the present invention and the scaffold protein.

In the method for obtaining antigen presenting cells, it is preferable to select the R1 portion of the difluoromethylaryl skeleton shown below, depending on the type of the scaffold protein.

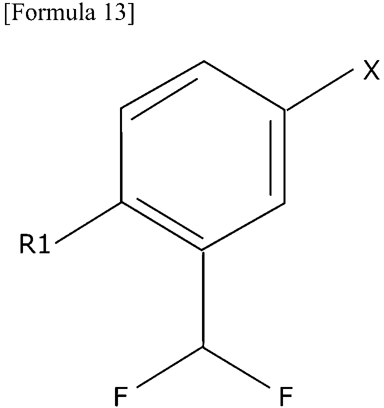

As an example, if Neuraminidase 1 (NEU1) or Neuraminidase 3 (NEU3) is used as a protein that serves as a scaffold in the cell membrane, a neuraminic acid group or a neuraminic acid derivative group can be selected as an R1 portion of the compound of the present invention. Based on the enzyme specificity of NEU1 or NEU3, a NEU1/NEU3 conjugate can be formed between NEU1 or NEU3 and the compound of the present invention. In this way, NEU1 or NEU3 functions as a scaffold in the cell membrane, so that the dynamic epitope and the carrier site (carrier protein portion) in the conjugate of the present invention can be presented to outside of the cell.

In the conjugate of the present invention having neuraminic acid or a neuraminic acid derivative as an R1 portion, the neuraminic acid or the neuraminic acid derivative portion functions as an enzyme activity site of NEU1 or NEU3. Furthermore, as a result of a nucleophilic reaction occurring between the F of the difluoromethylaryl group in the conjugate and the nucleophilic side chain of a glutamic acid or aspartic acid residue, etc. located within or near the enzyme active center of NEU1 or NEU3, either one F of the difluoromethylaryl skeleton can form an irreversible bond with NEU1 or NEU3. As a result, a linking structure consisting of NEU1 or NEU3 - a difluoromethylaryl skeleton-derived group - a dynamic epitope (DE) - a carrier site (CP; carrier protein) is formed on the cell surface (in the present description, such a structure is also referred to as a "NEU1/NEU3-conjugate"). The bond between NEU1 or NEU3 and the dynamic epitope (DE) is irreversible, and as a result, the dynamic epitope and the carrier protein that are derived from the conjugate of the present invention can be presented on the cell surface for a long period of time.

Based on the content described above, the present invention can provide, as another aspect, a method for forming a NEU1/NEU3-conjugate comprising NEU1 or NEU3, a dynamic epitope, and a carrier protein, by irreversibly binding a compound having the following formula with Neuraminidase 1 (NEU1) or Neuraminidase 3 (NEU3):

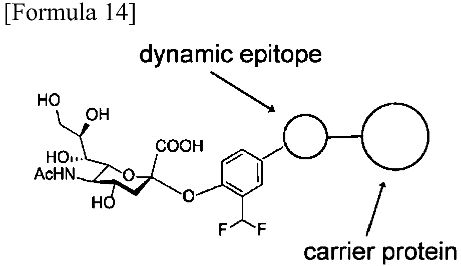

In the above formula, the dynamic epitope is selected from the group consisting of a protein or a peptide, a glycan, and a conjugate of a protein or a peptide and a glycan, and the carrier protein is a protein that exhibits an immunogenic action when it is combined with the dynamic epitope.

Since NEU1 or NEU3 exists as a membrane protein in the cell membrane, the compound of the present invention that binds to NEU1 or NEU3 is presented to outside of the cell membrane. The binding of NEU1 or NEU3 with the compound of the present invention can be generated as a result of a nucleophilic reaction occurring between the F of the difluoromethylaryl skeleton of the compound of the present invention and the nucleophilic side chain of a glutamic acid or aspartic acid residue, etc. located within or near the enzyme active center of NEU1 or NEU3.

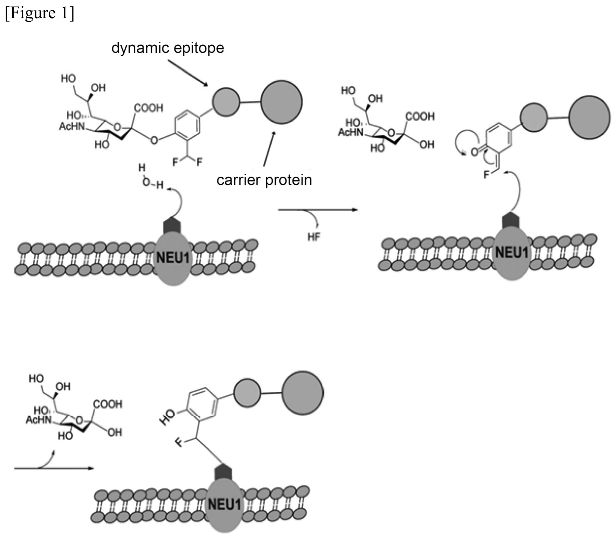

Specifically, the enzyme activity of NEU1 and/or NEU3 that exists as a membrane protein in the cell membrane is inhibited by a specific reaction mechanism using the compound of the present invention, and the compound of the present invention is allowed to irreversibly bind to NEU1 and/or NEU3, so that a NEU1/NEU3-conjugate comprising NEU1 and/or NEU3 and the dynamic epitope (and the carrier protein) bindng to the compound of the present invention can be formed. One specific example of this reaction (an example of LEU1) is shown in Figure 1.

Moreover, as another aspect of the present invention, there can be provided a method for obtaining antigen presenting cells, which present the antigenic site and the carrier site shown in the following formula (11) on the cell surface thereof, the method comprising allowing the conjugate represented by the formula (11) to come into contact with cells having Neuraminidase 1 (NEU1) or Neuraminidase 3 (NEU3):

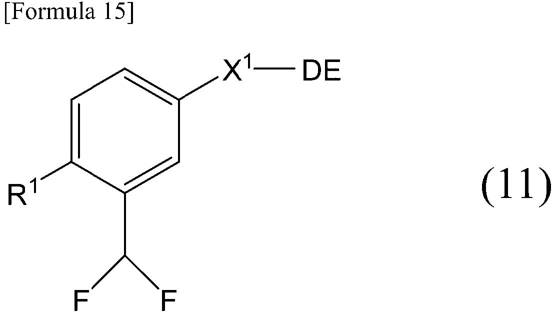

In the above formula (11), R¹ is a neuraminic acid group or a neuraminic acid derivative group, DE is an antigenic site selected from a glycoprotein and a glycopeptide, and X¹ and X² each independently represent a single bond or a linking group. The conjugate represented by the formula (11) has a neuraminic acid group or a neuraminic acid derivative group as R¹ that is linked to the difluoromethylaryl skeleton, and also has a dynamic epitope (DE) that is linked to the difluoromethylaryl skeleton.

In the present invention, the neuraminic acid or neuraminic acid derivative portion in the conjugate represented by the formula (11) functions as an enzyme activity site of Neuraminidase 1 (NEU1) or Neuraminidase 3 (NEU3) present on the surface of the cell membrane, and as a result of a nucleophilic reaction occurring between the F of the difluoromethylaryl group in the conjugate and the nucleophilic side chain of a glutamic acid or aspartic acid residue, etc. located within or near the enzyme active center of NEU1 or NEU3, either one F of the difluoromethylaryl skeleton can form an irreversible bond with NEU1 or NEU3. As a result, the antigenic site (DE) selected from a glycoprotein and a glycopeptide, which is presented by the formula (11), is linked to the cell surface. Thereby, cells that present the dynamic epitopes on the surface of the cell membrane (antigen presentation) can be obtained.

In the method for obtaining antigen presenting cells, it is preferable to select the R1 portion of the difluoromethylaryl skeleton shown below, depending on the type of the scaffold protein.

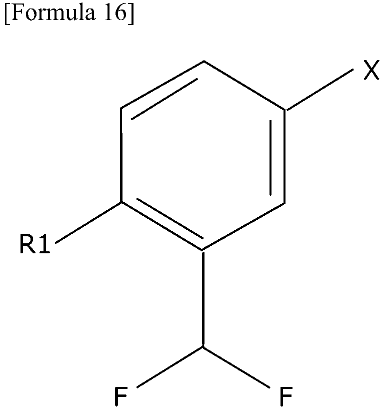

As an example, if Neuraminidase 1 (NEU1) or Neuraminidase 3 (NEU3) is used as a protein that serves as a scaffold in the cell membrane, a neuraminic acid group or a neuraminic acid derivative group can be selected as an R1 portion of the compound of the present invention. Based on the enzyme specificity of NEU1 or NEU3, a NEU1/NEU3 conjugate can be formed between NEU1 or NEU3 and the compound of the present invention. In this way, NEU1 or NEU3 functions as a scaffold in the cell membrane, so that the dynamic epitope and the carrier site (carrier protein portion) in the conjugate of the present invention can be presented to outside of the cell.

In the conjugate of the present invention having neuraminic acid or a neuraminic acid derivative as an R1 portion, the neuraminic acid or the neuraminic acid derivative portion functions as an enzyme activity site of NEU1 or NEU3. Furthermore, as a result of a nucleophilic reaction occurring between the F of the difluoromethylaryl group in the conjugate and the nucleophilic side chain of a glutamic acid or aspartic acid residue, etc. located within or near the enzyme active center of NEU1 or NEU3, either one F of the difluoromethylaryl skeleton can form an irreversible bond with NEU1 or NEU3. As a result, a linking structure consisting of NEU1 or NEU3 - a difluoromethylaryl skeleton-derived group - a dynamic epitope (DE) is formed on the cell surface (in the present description, such a structure is also referred to as a "NEU1/NEU3-conjugate"). The bond between NEU1 or NEU3 and the dynamic epitope (DE) is irreversible, and as a result, the dynamic epitope and the carrier protein that are derived from the conjugate of the present invention can be presented on the cell surface for a long period of time.

Utilizing such an action, the compound of the present invention is allowed to bind to target cells, in which NEU1 and NEU3 are present on the surface of the cell membrane, and as a result, the dynamic epitope and the carrier protein that bind to the compound of the present invention can be presented to the target cells. Herein, immune system cells, cancer cells, etc. can be selected as target cells.

The immune system cells that can be used as target cells may include immune system cells, such as activated macrophages, dendritic cells, and T cells. When the compound of the present invention is utilized to present the dynamic epitope to immune system cells, antibodies against the dynamic epitope can be generated.

The cancer cells that can be used as target cells may include any types of cancer cells, such as lung cancer, hepatocellular carcinoma, and colon cancer. By binding the compound of the present invention to NEU1 or NEU3 on cancer cells, existing antibodies that bind to the dynamic epitope can be delivered to the cancer cells. Such existing antibodies that bind to the dynamic epitope may be either antibodies that target cancer cells or antibodies that form antibody-drug conjugates (ADCs). The dynamic epitope can be selected, as appropriate, depending on the type of antibody used.

### (Method for generating antibody)

The conjugate of the present invention is preferably used for antibody generation. As another aspect, the present invention can provide a method for generating an antibody, comprising generating an antibody using antigen presenting cells, which present an antigenic site and a carrier site in the following formula (1) on the cell surface thereof:

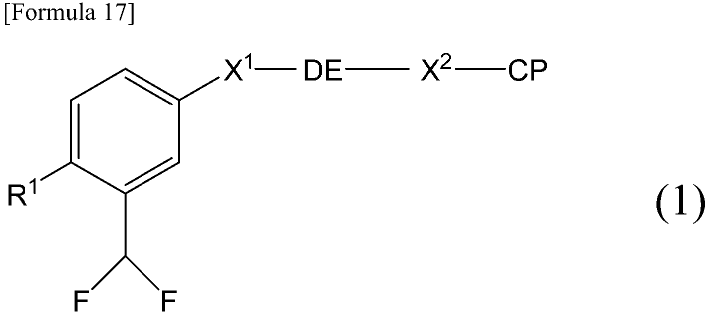

In the above formula (1), R¹ is neuraminic acid or a neuraminic acid derivative, DE is an antigenic site selected from a glycoprotein and a glycopeptide, CP is a carrier site, and X¹ and X² each independently represent a single bond or a linking group.

In method for generating an antibody, the antigen presenting cells produced by the aforementioned method are presented to immune system cells such as activated macrophages, dendritic cells, or T cells. Since the conjugate of the present invention has such a structure as shown in the above formula (1), it can present an antigen on the cell surface by an MHC independent method that does not depend on the binding with MHC molecules. Thus, the antigen that are presented to immune system cells has glycans, and a monoclonal antibody simultaneously recognizing both the glycans and peptides that constitute a glycopeptide can be obtained. It is to be noted that, in the present invention, it is also possible to obtain a monoclonal antibody that recognizes only the glycans in antigen-presented glycoprotein and glycopeptide.

Based on the content described above, the present invention can provide, as another aspect, a method for generating an antibody against a dynamic epitope, wherein the method comprises: irreversibly binding a conjugate having a formula shown below with Neuraminidase 1 (NEU1) or Neuraminidase 3 (NEU3), to form a conjugate comprising NEU1 or NEU3, a dynamic epitope, and a carrier protein; and presenting the dynamic epitope and the carrier protein in the conjugate to immune system cells:

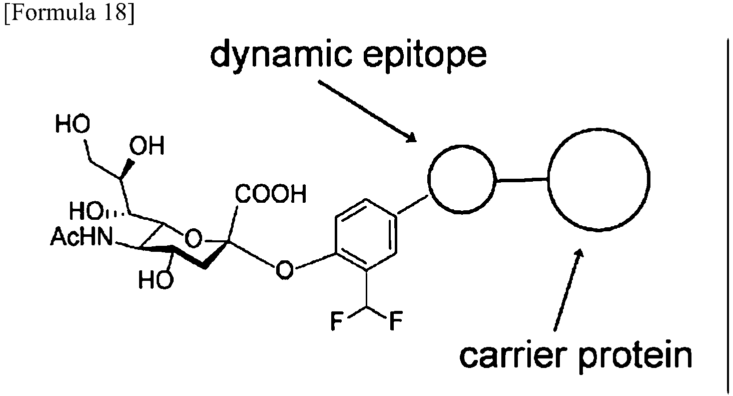

In the above formula, the dynamic epitope is selected from the group consisting of a protein or a peptide, a glycan, and a conjugate of a protein or a peptide and a glycan, and the carrier protein is a protein that exhibits an immunogenic action when it is combined with a dynamic epitope.

In the present invention, as immune system cells that are to be presented a dynamic epitope and a carrier protein by the compound of the present invention, immune system cells such as activated macrophages, dendritic cells, and T cells can be used.

### (Intended use)

The conjugates represented by the formulae (1) and (11) are preferably used to bind to cells having a protein serving as a scaffold, as described later. Preferred examples of the protein serving as a scaffold may include Neuraminidase 1 (NEU1) and Neuraminidase 3 (NEU3). That is to say, the conjugate of the present invention is preferably used to bind to cells having Neuraminidase 1 (NEU1) or Neuraminidase 3 (NEU3).

Among others, the conjugate represented by the formula (1) is preferably used for antibody generation. As described later, by binding the conjugate to cells having Neuraminidase 1 (NEU1) or Neuraminidase 3 (NEU3) as a protein serving as a scaffold on the surface of the cell membrane thereof, antigen presenting cells, which present an antigenic site and a carrier site on the cell surface, can be obtained. Then, the antigen presenting cells present the antigen to immune system cells, such as activated macrophages, dendritic cells, and T cells, so that an antibody specific to an antigen (dynamic epitope) having a glycan can be generated.

Furthermore, the conjugates represented by formulae (1) and (11) are also useful as medicaments for treating tumors and/or cancers. It has been known that Neuraminidase 1 (NEU1) and/or Neuraminidase 3 (NEU3) are highly expressed on the surface of tumor and/or cancer cells (target cells). Therefore, for example, by introducing an antigenic site, for which an antibody has already been known, into the conjugate of the present invention and then allowing the conjugate to come into contact with target cells in which Neuraminidase 1 (NEU1) and/or Neuraminidase 3 (NEU3) are highly expressed, the antigenic site and the carrier site can be presented on the surface of the target cells. Then, by allowing the antibody to act on antigen presenting cells, the antibody binds to the target cells or enters the cells, thereby repelling the target cells. It is to be noted that the antibody may also be an antibody that forms an antibody-drug conjugate (ADC).

### (Pharmaceutical composition/vaccine)

The present invention can also provide, as another aspect, a pharmaceutical composition comprising the aforementioned conjugate. The conjugate of the present invention can be directly administered alone, but it may also be administered after mixing with pharmaceutically acceptable formulation components. The pharmaceutical composition of the present invention can be used for animals and humans.

The pharmaceutical composition may be, for example, a vaccine. In other words, the present invention can provide, as another embodiment, a vaccine comprising the aforementioned conjugate.

With regard to the route of administration of the pharmaceutical compositions, it is preferable to use an administration route that is the most effective for the treatment, and the administration route may be oral administration, or parenteral administration such as nasal, rectal, buccal, subcutaneous, intramuscular, or intravenous administration. Possible administration forms may include capsules, tablets, granules, powders, syrups, emulsions, suppositories, and injections. Liquid preparations suitable for oral administration, such as emulsions and syrups, can be produced using water, sugars such as sucrose, sorbitol and fructose, glycols such as polyethylene glycol and propylene glycol, oils such as sesame oil, olive oil and soybean oil, antiseptics such as p-hydroxybenzoic acid esters, and flavors such as strawberry flavor and peppermint. Moreover, capsules, tablets, powders, granules, etc. can be produced using excipients such as lactose, glucose, sucrose and mannit, disintegrants such as starch and sodium alginate, lubricants such as magnesium stearate and talc, binders such as polyvinyl alcohol, hydroxypropyl cellulose and gelatin, surfactants such as fatty acid esters, and plasticizers such as glycerin.

The formulation suitable for parenteral administration preferably consists of a sterile aqueous formulation comprising an active compound that is isotonic with the blood of a recipient. For example, in the case of an injection, a solution for injection is prepared using a carrier, etc. consisting of a saline solution, a glucose solution, or a mixture of a saline solution and a glucose solution. In addition, even in the case of a parenteral agent, one or more auxiliary ingredients selected from the glycols, oils, flavors, antiseptics (including antioxidants), excipients, disintegrants, lubricants, binders, surfactants, plasticizers, etc., exemplified for oral agents, may be added to the parenteral agent.

The pharmaceutical composition of the present invention may be used for the prevention and/or treatment of tumors and/or cancers. For example, by administration of the pharmaceutical composition of the present invention, an antibody that prevents and/or treats tumors and/or cancers can be generated.

Moreover, the pharmaceutical composition of the present invention may also be a combination preparation comprising a first agent containing the aforementioned conjugate and a second agent containing an antibody that binds to the antigenic site of the aforementioned conjugate. For example, by introducing an antigenic site, for which an antibody has already been known, into the conjugate of the present invention and then allowing the conjugate to come into contact with target cells in which Neuraminidase 1 (NEU1) and/or Neuraminidase 3 (NEU3) are highly expressed, the antigenic site and the carrier site can be presented on the surface of the target cells. Then, by allowing the antibody to act on antigen presenting cells, the antibody binds to the target cells or enters the cells, thereby treating tumors and/or cancer.

The effective dose and the frequency of administration of the pharmaceutical composition vary depending on the structure of the conjugate, the dosage form, the patient's age and weight, the nature or severity of the symptoms to be treated, and the like, and thus, the effective dose and the frequency of administration are determined by comprehensively considering these factors. Furthermore, the higher the binding ability of the pharmaceutical composition to Neuraminidase 1 (NEU1) or Neuraminidase 3 (NEU3), the more the medicinal effects that can be exhibited even at a low dose.

### (Method for treating tumor and/or cancer)

The present invention can provide, as another aspect, a method for treating a tumor and/or a cancer, comprising administering a conjugate, and administering an antibody binding to the antigenic site in the above formula (1) or formula (11). However, the treatment method of the present invention may exclude a method for treating humans in some cases. In addition, another aspect of the present invention relates to use of the aforementioned conjugate for production of a preventive and/or therapeutic agent for tumors and/or cancers.

It has been known that Neuraminidase 1 (NEU1) and/or Neuraminidase 3 (NEU3) are highly expressed on the surface of tumor and/or cancer cells (target cells). Therefore, for example, by introducing an antigenic site, for which an antibody has already been known, into the conjugate of the present invention and then allowing the conjugate to come into contact with target cells in which Neuraminidase 1 (NEU1) and/or Neuraminidase 3 (NEU3) are highly expressed, the antigenic site can be presented on the surface of the target cells. Then, by allowing the antibody to act on antigen presenting cells, the antibody binds to the target cells or enters the cells, thereby treating tumors and/or cancer. It is to be noted that the antibody may also be an antibody that forms an antibody-drug conjugate (ADC).

The present invention can also provide, as another aspect, a method of inducing an antibody against a dynamic epitope by irreversibly binding the compound of the present invention to Neuraminidase 1 (NEU1) or Neuraminidase 3 (NEU3) present on the cell membrane of cancer cells to form a conjugate comprising NEU1 or NEU3 and a dynamic epitope, and then by binging thereto an existing antibody that specifically binds to the dynamic epitope in the conjugate.

By utilizing the compound of the present invention, therapeutic antibodies can be efficiently induced to cancer cells, or therapeutic agents bound to antibodies can be efficiently induced to cancer cells, even when existing antibodies have low specificity for cancer cells and cannot effectively treat the cancer cells, or when there are no therapeutic antibodies specific to a specific cancer cell type, or in other cases.

### Examples

Hereinafter, the present invention will be specifically described in the following examples. The following examples are not intended to limit the present invention by any method.

### Example 1: Synthesis of terminal compound

(1-1) Compound 8 (NEU1 Sialidase Inhibitor Derivative 8) was synthesized according to the reaction scheme shown below.

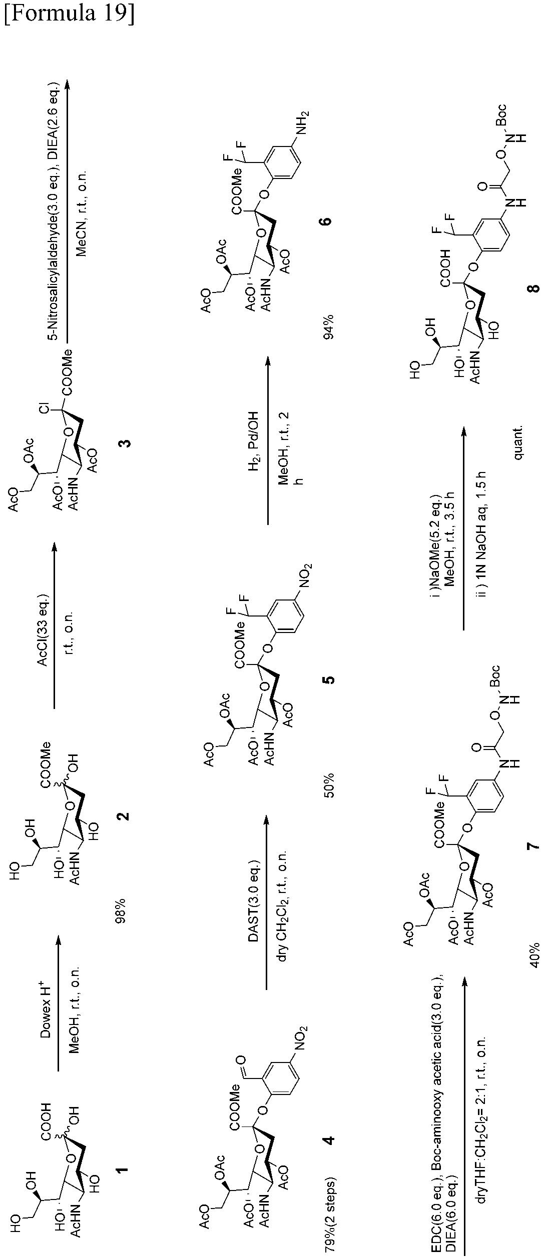

Compound 8, a compound that specifically and easily reacts with a compound having ketone/aldehyde groups in an aqueous solution under mild weakly acidic conditions, can be synthesized by the methods disclosed in the previous reports (e.g., WO2017/131242, Nishimura, S.-I. et al., Characterization of Vibrio cholerae neuraminidase by novel mechanism-based fluorescent labeling reagent. Biochemistry 2005, 44, 11669-11675). Specifically, Compound 8 was synthesized by condensing the above-described Compound 6 with Boc-aminooxy acetic acid, followed by deprotection of the acetyl group and the hydrolysis of the methyl ester.

¹H NMR (600 MHz, D₂O) δ ppm 7.66 (s, 1H), 7.47 (d, 1H), 7.32 (d, 1H), 7.00 (t, 1H), 4.42 (s, 2H), 3.90 (t, 1H), 3.86 (t, 1H), 3.75 (m, 3H), 3.55 (dd, 1H), 3.51 (d, 1H), 2.79 (dd, 1H), 1.95 (s, 3H), 1.94 (dd, 1H), 1.36 (s, 9H) , MALDI-TOFMS: m/z calcd for C₂₅H₃₅F₂N₃O₁₃ [M+Na]⁺ 646.203, found 646.437.
(1-2) Compound 9 (NEU1 Sialidase Inhibitor Derivative 9) was synthesized according to the reaction scheme shown below.

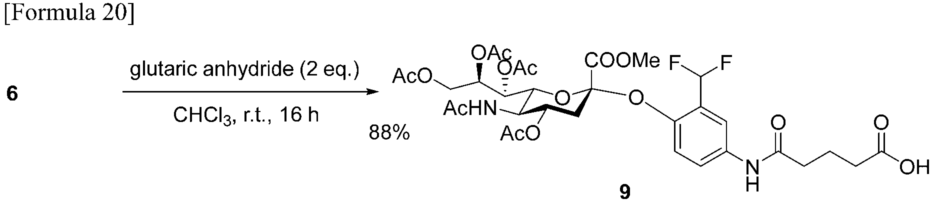

Compound 9, which is directly coupled and introduced as a cap into the N-terminus in the final step of solid-phase synthesis when a peptide, a glycopeptide, etc. is presented as an antigen, was obtained by the reaction of the Compound 6 with glutaric acid anhydride.

¹H NMR (600 MHz, CDCl₃) δ ppm 7.93 (s, 1 H, CONH), 7.83 (s, 1 H, Ar), 7.56 (d, J=9.1 Hz, 1 H, Ar), 7.24 (d, J=8.7 Hz, 1 H, Ar), 6.90 (t, J=55.2 Hz, 1 H, CHF₂), 5.46 (d, J=10.2 Hz, 1 H, NHAc), 5.38 (m, 2 H, H-7, H-8), 4.98 (td, J=11.1, 4.2 Hz, 1 H, H-4), 4.44 (d, J=10.6 Hz, 1 H, H-6), 4.31 (dd, J=12.4, 1.8 Hz, 1 H, H-9), 4.13 (m, 2 H, H-5, H-9), 3.67 (s, 3 H, COOMe), 2.75 (dd, J=12,8, 4.5 Hz, 1 H, H-3_{eq}), 2.50 (m, 4 H, 2×linker CH₂), 2.24 (t, J=12.5 Hz, 1 H, H-3ₐₓ), 2.16 (2×s, 6 H, 2×Ac), 2.07 (m, 8 H, 2×Ac, linker CH₂), 1.94 (s, 3 H, Ac).

¹³C NMR (150 MHz, CDCl₃) δ ppm 123.02, 120.38, 117.43 (Ar), 73.24 (C-6), 68.73 (C-8), 68.31 (C-4), 66.94 (C-7), 61.80 (C-9), 52.99 (COOMe), 49.21 (C-5), 37.69 (C-3), 35.90 (linker CH₂), 32.54 (linker CH₂), 22.95, 20.96, 20.79 (Ac), 20.01 (linker CH₂).

MALDI-TOFMS: m/z calcd for C₃₂H₄₀F₂N₂O₁₆ [M+Na]⁺ 769.224, found 769.207.

### Example 2: Synthesis of dynamic epitope (antigenic site)

The antigen glycopeptide of a SARS-CoV-2 spike glycoprotein was synthesized as a dynamic epitope (antigenic site).
(2-1) According to the reaction scheme shown below, Glycopeptide Precursor 10 having a 5-oxohexanoic acid residue was synthesized.

Glycopeptide Precursor 10: 5-oxohexanoic acid-³³⁴Gln-Val-Ser-Pro-Phe-Gly-Glu-Val-Phe-Asn (Fucα1,6GlcNAcβ1)-Ala-Thr-Arg-Phe-Ala-Ser-Val³⁵⁰-Cys-NH₂ (SEQ ID NO: 1)

Precursor 10 of the glycopeptide antigen (Compound 11 shown below) in the RBD of the SARS-CoV-2 spike glycoprotein was synthesized by a Fmoc solid-phase peptide synthesis method. Basically, using Rink Amide-ChemMatrix resin (0.42 mmol/g, 100 mg, 42 µmol), individual steps were sequentially proceeded by performing a reaction for 10 minutes for each step, under microwave irradiation (50°C) in a DMF solution consisting of a Fmoc amino acid derivative (168 µmol), HBTU (168 µmol) and HOBt (168 µmol). Unreacted amino acids were acetylated by treating them using a solution of acetic anhydride/DIEA/DMF (v : v : v = 0.5 : 1 : 8.5) at room temperature for 30 seconds. The Fmoc groups were deprotected by treating them with 20% piperidine/DMF for 3 minutes under microwave irradiation (50°C).

The peptide of interest was synthesized by repeating the aforementioned three steps: (A) elongation of various types of Fmoc amino acids, (B) acetylation of unreacted amino groups, and (C) de-Fmocation. However, at the position of Asn³⁴³, instead of the usual Fmoc amino acid derivative, an Fmoc glycoamino acid derivative, to which a disaccharide consisting of fucose and N-acetylglucosamine bound, was separately produced by enU Pharma, Inc., and the synthetic reaction was proceeded using this Fmoc glycoamino acid derivative.

After condensation of all amino acids and glycoamino acids, 5-oxohexanoic acid was condensed to the N-terminal amino group under the same conditions as those for Fmoc amino acid elongation. Furthermore, trifluoroacetic acid : water : phenol : thioanisole : 1-dodecanethiol (v : v : v : v : v = 82.5 : 5 : 5 : 5 : 2.5) were added to the reaction system, and the obtained mixture was then shaken at room temperature for 2 hours, so that a synthetic intermediate of interest was cleaved from the solid-phase carrier, and at the same time, the side chain was deprotected.

The reaction solution was filtrated, tert-butylmethyl ether was then added, and the obtained mixture was then centrifuged (5000 G, 4°C, 10 minutes). The supernatant was discarded, and the precipitate was dissolved in a 50% acetonitrile aqueous solution, and was then freeze-dried to obtain a crude product of Glycopeptide Precursor 10 of interest. This freeze-dried product was dissolved in methanol, and a 1 N sodium hydroxide aqueous solution was then added thereto to adjusted the pH to pH 12.5. The resulting solution was shaken at room temperature for 1 hour to deprotect acetyl groups in the glycan region. Thereafter, a 30% acetic acid aqueous solution was used to return the solution to neutral, and the solvent was then removed under reduced pressure conditions. The residue was purified by reversed-phase high performance liquid chromatography to obtain Glycopeptide Precursor 10 of interest.

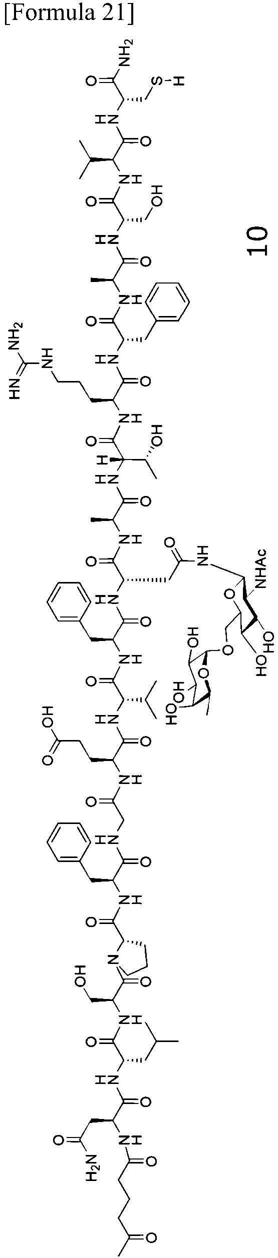

MALDI-TOFMS: m/z calcd for C₁₀₈H₁₆₃N₂₄O₃₇S [M+H]+ 2420.133, found 2420.168.
(2-2) Subsequently, Glycopeptide Antigen 11 was synthesized as follows. An oxazoline-sialylglycan derivative (5 mM) prepared according to a known method (Shoda, S.-i. et al., J. Org. Chem. 2009, 74, 2210-2212) and Endo-F3 (100 U/mL) were left at rest in 100 mM HEPES (pH 7.0) at 37°C for 4 hours, so that they were allowed to react with the aforementioned Glycopeptide Precursor 10 (1 mM). The reaction mixture was purified by reversed-phase high-performance liquid chromatography to obtain Glycopeptide Antigen 11 as freeze-dried powders (yield: 26.2%).

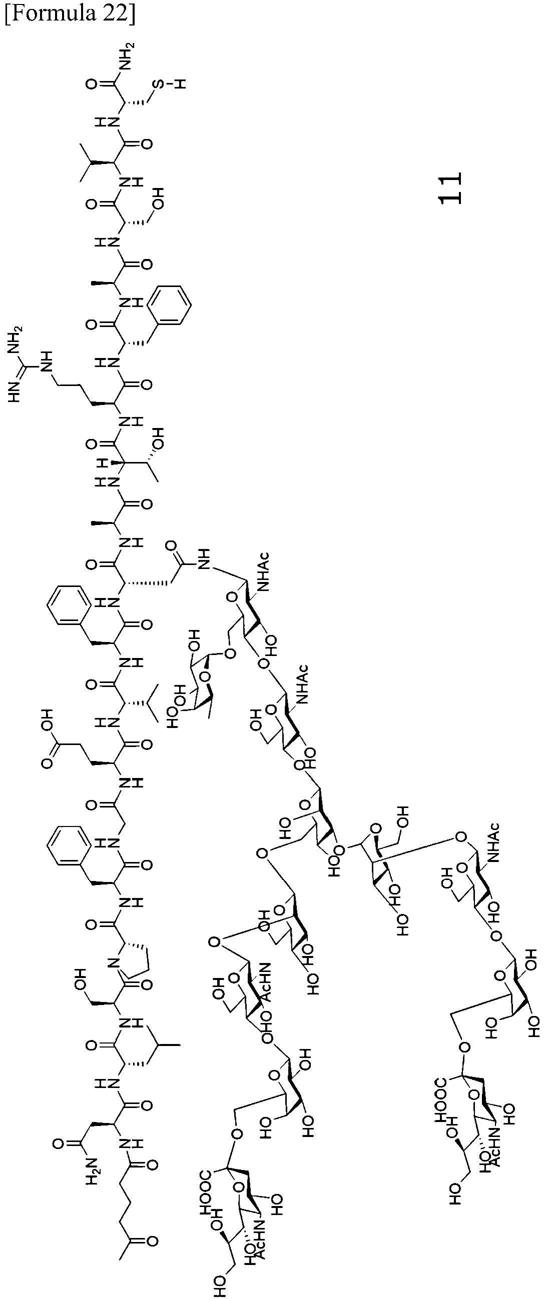

MALDI-TOFMS: m/z calcd for C₁₈₄H₂₈₄N₂₉O₉₃S [M-H]- 4419.8181, found 4418.978
In addition, the following non-glycosylated peptide (SEQ ID NO: 2) used in the screening of antibodies was also synthesized in the same manner as described above.

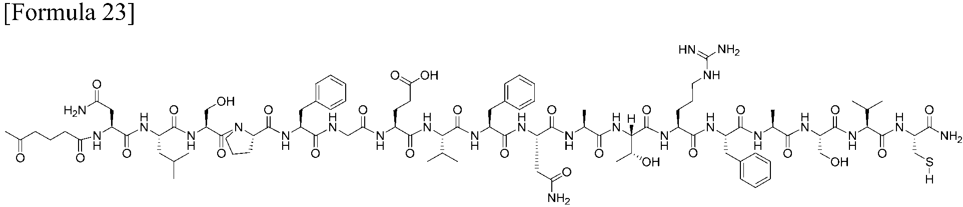

MALDI-TOF MS: m/z calcd for C₉₄H₁₄₀N₂₃O₂₈S [M+H]+ 2070.991 ,found 2070.843.

### Example 3: Conjugation of dynamic peptide (antigen) and terminal compound

Conjugate 12 was synthesized by conjugating the Compound 8 (NEU1 Sialidase Inhibitor Derivative 8) synthesized in Example 1 with the Glycopeptide Antigen 11, the dynamic peptide synthesized in Example 2.

The Compound 8 (NEU1 Sialidase Inhibitor Derivative 8) (50 mM) synthesized in Example 1 left at rest in a 1 N hydrochloric acid aqueous solution at 40°C for 1 hour. Thereafter, the solution was neutralized by addition of an ammonium bicarbonate aqueous solution. The sialidase inhibitor, which was deprotected by HPLC to expose the aminooxy groups, was purified and freeze-dried, and was then promptly coupled with the Glycopeptide Antigen 11 synthesized in Example 2.

The synthesis reaction was allowed to proceed, while the glycopeptide (final concentration: 1 mM) and the deprotected sialidase inhibitor (final concentration: 20 mM) were dissolved in a 100 mM acetate buffer (pH 4) and the obtained solution was then concentrated by centrifugation over 16 hours. Thereafter, the reaction product was purified by HPLC to obtain Conjugate 12.

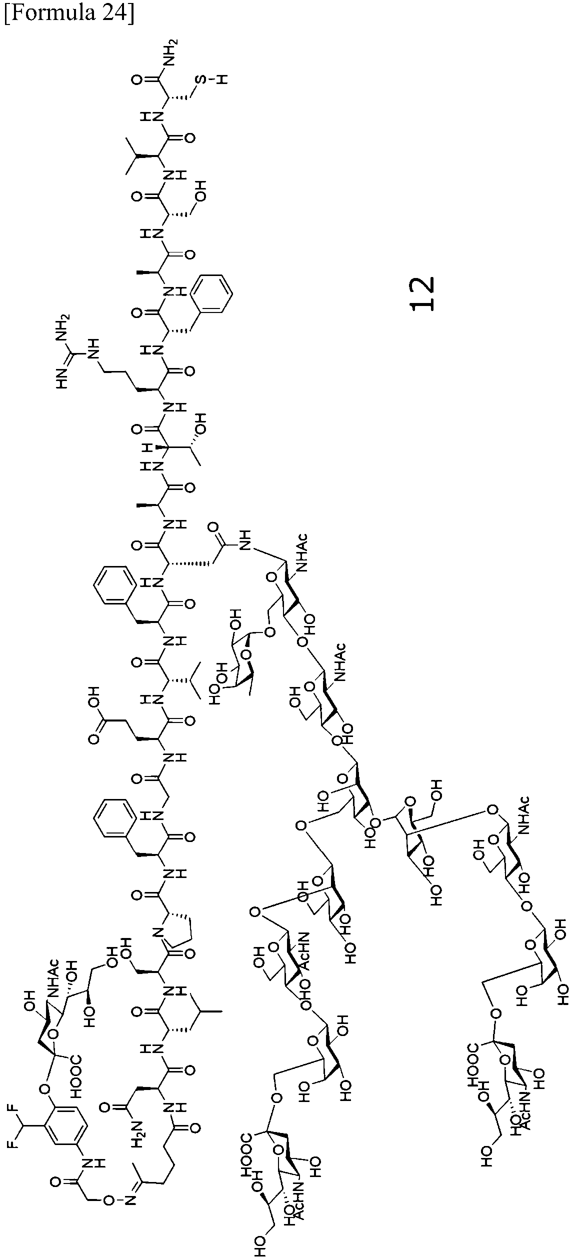

MALDI-TOF MS: m/z calcd for C₂₀₄H₃₀₉F₂N₃₂O₁₀₃S [M-H]- 4924.969, found 4924.930

### Example 4: Antibody generation using conjugate

Conjugate 12 produced in Example 3 was conjugated to KLH (carrier protein) according to a maleimidobenzoyloxysuccinimide (MBS) method to obtain a conjugate for use in antibody generation. Approximately 0.1 mg of the obtained conjugate for use in antibody generation was administered subcutaneously to the base of the tail of three mice (B6D2F1/Slc), so that an immune response was provoked. A booster immunization was administered, 17 days after the subcutaneous administration, by the same method as described above. Thereafter, blood was collected 4 days after, and iliac lymph nodes were then harvested. Lymphocytes collected from the harvested iliac lymph nodes were fused with myeloma cells SP2, and were then cultured in a HAT selection medium to select antibody-producing cells.

Next, the culture supernatant of the resulting hybridoma was seeded on an ELISA plate. Positive wells were selected based on binding to a bovine serum albumin (BSA)-glycopeptide antigen conjugate (BSA-MBS-glycopeptide (19-1) conjugate) and binding to a BSA-peptide antigen conjugate (a conjugate of BSA and the non-glycosylated peptide prepared in Example 2) (BSA-MBS-unmodified peptide (19-1) conjugate). The positive wells were screened based on a binding reaction with the glycopeptide that was a dynamic epitope and the non-glycosylated peptide that was a control.

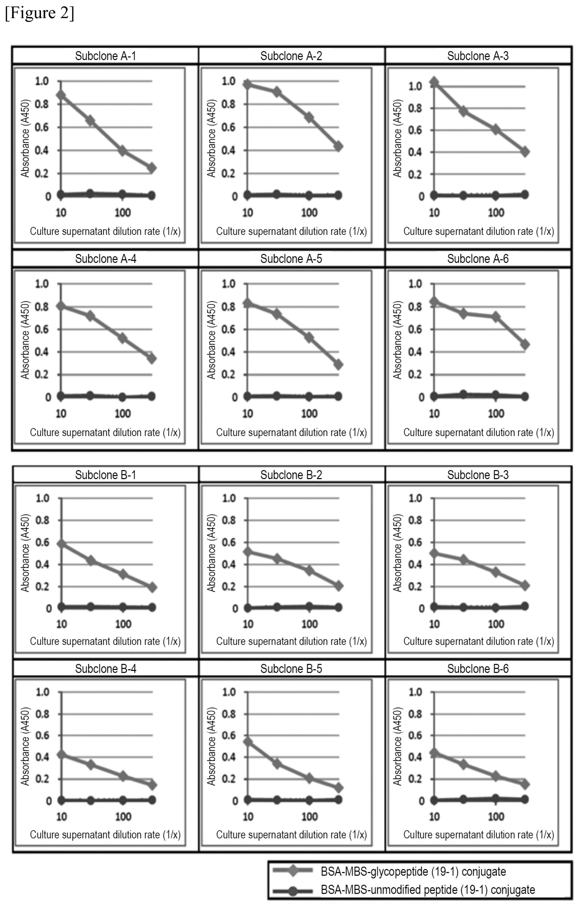

Fused cells were cloned by a limiting dilution method, etc. Figure 2 shows the binding specificity of the culture supernatants of each 6 types of single clones (subclone A-1 to subclone A-6 and subclone B-1 to subclone B-6) obtained from two clone lines, clone A and clone B, to the glycopeptide (dynamic epitope). These results have demonstrated that the antibodies obtained from the culture supernatants of these clones bound only to the glycopeptide, and that these antibodies do not exhibit affinity for non-glycosylated peptide.

### Example 5: Evaluation of binding specificity of monoclonal antibody to SARS-CoV-2 spike glycoprotein

The binding specificity of the monoclonal antibody against the SARS-CoV-2 spike glycoprotein produced in Example 4 to the SARS-CoV-2 spike glycoprotein was examined.

A PBS solution (100 µL), in which a SARS-CoV-2 S1 protein at a concentration of 1 µg/mL had been dissolved, was added to the wells of a nonspecific adsorption plate for ELISA (Sumitomo Bakelite Co., Ltd.), and was then left at rest at 4°C overnight. Thereafter, the solution was discarded, and the wells were washed with PBS. After that, 200 µL of PBS containing 1% BSA was added to the wells, and was then left at rest at room temperature for 2 hours to block the surface. After discarding the solution and washing the wells, each culture supernatant was diluted 10-fold with an ELISA buffer (25 mM Tris, 150 mM NaCl, 0.05% Tween-20, 0.1% BSA, pH 7.5), and 100 µL of each diluted culture supernatant was added to the plate, and was left at rest at room temperature for 2 hours. Thereafter, the wells were washed in the same manner as described above, and 100 µL of anti-mouse IgG antibody AP conjugate (Promega) that had been diluted to 1/2500 with the ELISA buffer was added, and was then left at rest at room temperature for 1 hour.

The solution was discarded, and the wells were washed with PBS to completely remove the secondary antibody, and thereafter, a 4-nitrophenol phosphate solution was added, and the wells were incubated at room temperature for 2 hours. The absorbance at 405 nm was measured using a plate reader.

The results are shown in Figure 3. It was demonstrated that both of the antibodies obtained from the culture supernatants of clone A and clone B strongly bind to the spike glycoprotein (S1 WT) of the SARS-CoV-2 wild strain and the spike glycoprotein (S1 Delta) of the Delta strain (Figure 3(A) and Figure 3(B)). Furthermore, it became clear that subclone A-4 and subclone A-2 also strongly bind to the RBD of the recombinant spike glycoprotein of the Omicron strain (BA.2.75), to which Sotrovimab cannot bind (Figure 3(C)).

### Example 6: Evaluation of the binding affinity of monoclonal antibodies to SARS-CoV-2 spike glycoprotein

The binding affinity of the monoclonal antibodies against a SARS-CoV-2 spike glycoprotein produced in Example 4, to the SARS-CoV-2 spike glycoprotein, was examined.

Recombinant SARS-CoV2 S1-mFc (Sino Biological) was immobilized on an SPR sensor chip CM5 using an amine coupling method. Using Biacore 2000, the dissociation constants between SARS-CoV2 S1-mFc and monoclonal antibody subclone A-2 and subclone B-3, which had been purified with a Spin Column-Based Antibody Purification Kit (Protein G) (Thermo Fisher Scientific), were calculated.

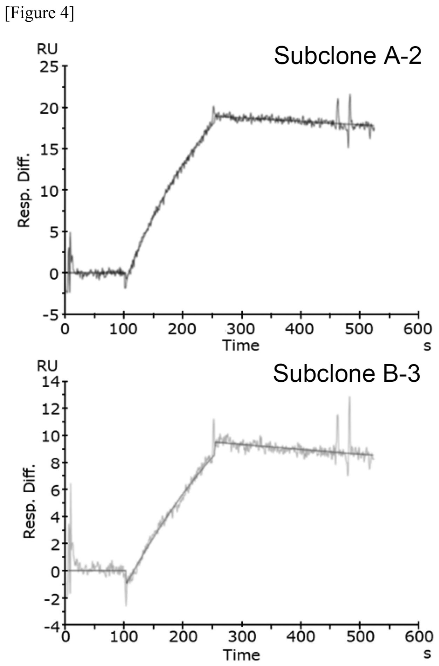

The results are shown in Figure 4. The dissociation constants were Kd = 7.4 nM for subclone A-2 and Kd = 17 nM for subclone B-3 (Figure 4). These results demonstrate that the monoclonal antibody clones produced using the conjugate of the present invention strongly bind to the spike glycoprotein (S1 WT) of the SARS-CoV-2 wild strain at the order of nM.

### Example 7: Verification of the significance and necessity of conjugation with NEU1 sialidase irreversible inhibitor

In the present invention, a conjugate having an antigenic site is allowed to covalently bind to NEU1 sialidase on immune cells or cancer cells, thereby presenting antigens on the cell surface and generating antibodies. With regard to various antibodies acquired by conventional techniques, some antibodies have shown a preference for glycopeptides in some cases, most have been reported to also exhibit strong binding affinity to main chain peptides lacking glycans (e.g., Corzana, F. et al., Chem. Soc. Rev. 2017, 46, 7154-7175). In other words, using conventional techniques, it had been difficult to generate antibodies having high specificity to glycopeptides having glycans.

The purpose of the present example is to conduct comparative experiments for demonstrating the significance and necessity of conjugating an antigenic site with NEU1 sialidase irreversible inhibitors. A glycopeptide antigen-KLH conjugate unconjugated with a NEU1 sialidase irreversible inhibitor was prepared using a conventional method, and an attempt was made to obtain an antibody.

A glycopeptide (SEQ ID NO: 3) containing two glycosylated sites, Thr323 and Ser325, of the SARS-CoV-2 spike glycoprotein and having a 5-oxohexanoic acid residue at the terminus, and a non-glycosylated peptide (SEQ ID NO: 4) were synthesized using Fmoc-Thr [Ac4Galβ(1 → 3)Ac2GalNAcα1 → ]-OH and Fmoc-Ser [Ac4Galβ(1 → 3)Ac2GalNAcα1→]-OH according to the solid-phase synthesis protocols of Compound 10.

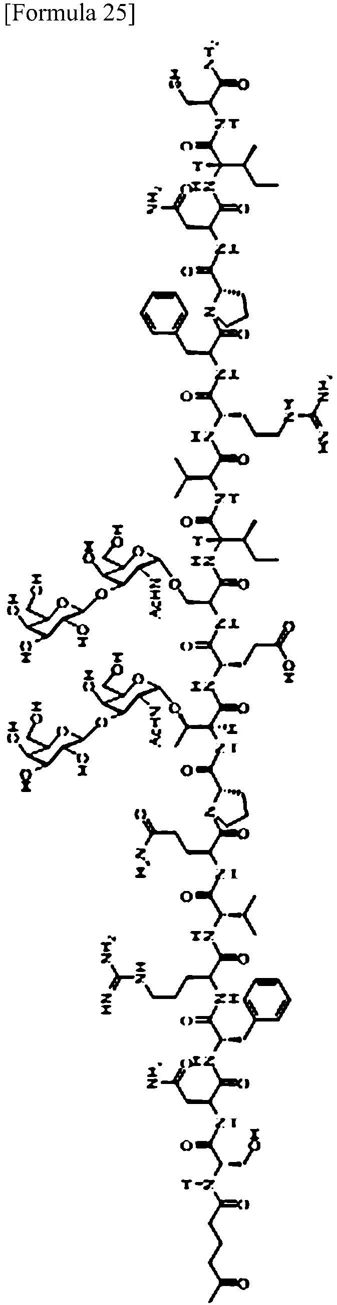

MALDI-TOF/MS: m/z calcd for C₁₂₇H₂₀₃N₃₀O₄₈S [M+H]+ 2948.404, found 2948.516

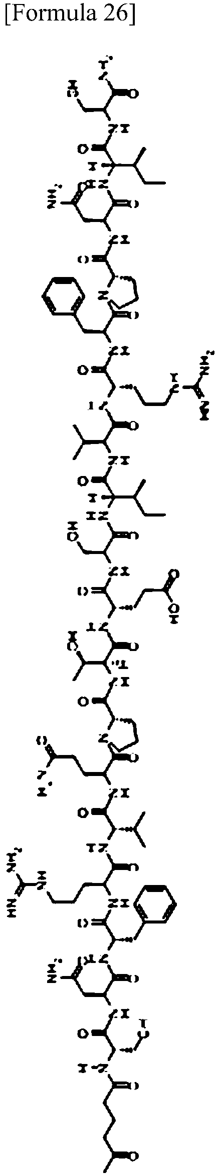

MALDI-TOF/MS: m/z C₉₉H₁₅₆N₂₈O₂₈S [M+H]+ 2218.140, found 2218.112
Glycopeptide antigen and non-glycosylated peptide antigen, which had not been conjugated with the above-described NEU1 sialidase irreversible inhibitor, were conjugated to KLH according to a maleimidobenzoyloxysuccinimide (MBS) method to obtain conjugates. Approximately 0.1 mg of the obtained conjugate was administered subcutaneously to the base of the tail of three mice (B6D2F1/Slc), so that an immune response was provoked. A booster immunization was administered, 17 days after the subcutaneous administration, by the same method as described above. Thereafter, blood was collected 4 days after, and iliac lymph nodes were then harvested. Lymphocytes collected from the harvested iliac lymph nodes were fused with myeloma cells SP2, and were then cultured in a HAT selection medium to select antibody-producing cells.

Next, the culture supernatant of the resulting hybridoma was seeded on an ELISA plate. Positive wells were selected based on binding to a BSA-glycopeptide antigen conjugate and binding to a BSA-peptide antigen conjugate. The positive wells were screened based on a binding reaction with the glycopeptide that was a dynamic epitope and the non-glycosylated peptide that was a control.

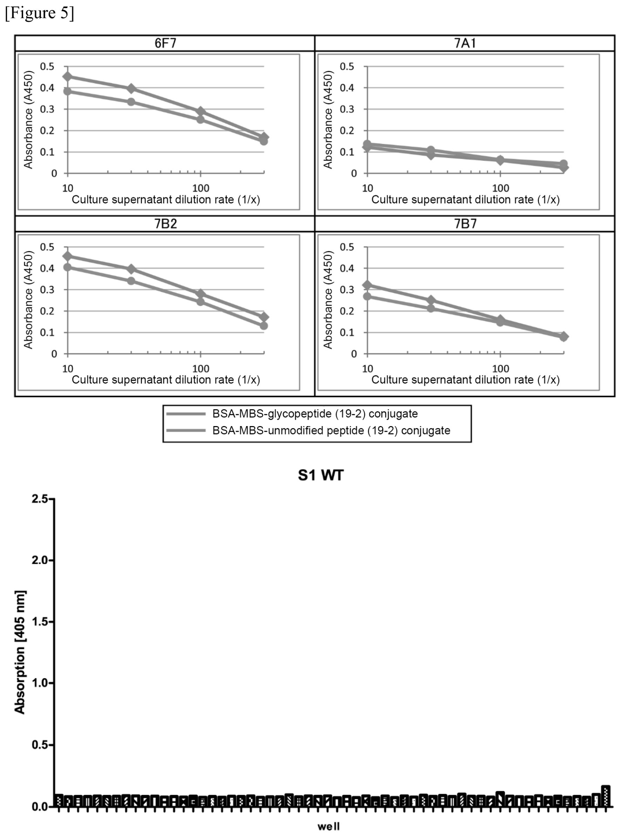

Figure 5 shows representative examples of the screening results obtained using antibodies produced using glycopeptide antigen-KLH conjugates by conventional methods, along with the reactivity of these antibodies with spike glycoprotein (S1) derived from the SARS-CoV-2 wild strain. The antibodies produced by conventional methods could not recognize the glycans of the antigen (Figure 5A) and did not react, either, with the wild-type spike protein (S1 WT) (Figure 5B; in the figure, clone names omitted).

The glycopeptide antigen-KLH conjugate that was not conjugated with a NEU1 sialidase irreversible inhibitor was not presented extracellularly and was incorporated into dendritic cells, etc., and was then decomposed into antigen peptides, which were then presented on the cell surface by major histocompatibility complex (MHC) class II molecules. Therefore, the glycopeptide antigen-KLH conjugate that is not conjugated with a NEU1 sialidase irreversible inhibitor is not subjected to antigen presentation in the state it has glycans and as a result, an antibody specifically binding to the dynamic epitope having glycans is not generated (Figure 5). In contrast, in the present invention, a NEU1 sialidase irreversible inhibitor-glycopeptide antigen-KLH conjugate, which is formed by conjugation with a NEU1 sialidase irreversible inhibitor, is used, so that the antigen can be presented extracellularly without passing through the pathway of antigen peptide presentation by MHC molecules (i.e., MHC-independent antigen presentation). Thereby, the antigen is presented extracellularly without decomposition of the glycans of the glycopeptide, etc., and thus, an antibody that specifically binds to the dynamic epitope containing glycans can be generated.

### Example 8: Antigen (target molecule) presentation on surface of cancer cells

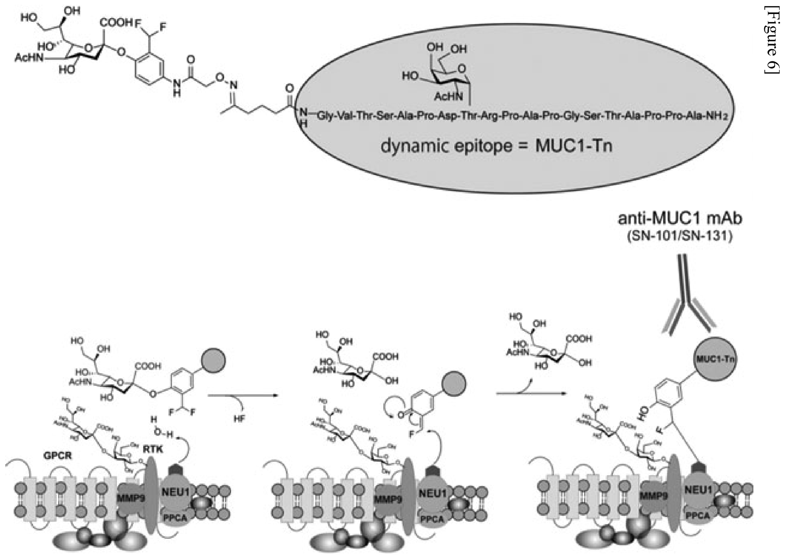

An experiment was conducted for the purpose of examining whether or not a dynamic epitope (target molecule) that serves as a binding target of an anti-MUC1 antibody can induce the anti-MUC1 antibody to cancer cells by allowing the dynamic epitope to bind to NEU1 sialidase present on the surface of the cancer cells. The scheme for inducing the antibody in this example is shown in Figure 6.
(8-1) Conjugation of Compound 8 with dynamic epitope of anti-MUC antibody (synthesis of Compound 13)

The Compound 8 (50 mM) synthesized in Example 1 was left at rest in a 1 N hydrochloric acid aqueous solution at 40°C for 1 hour. Thereafter, the solution was neutralized by addition of an ammonium bicarbonate aqueous solution. Compound 8, which was deprotected by HPLC to expose the aminooxy group, was purified and was then freeze-dried. After the freeze-drying, MUC1-Tnglycopeptide (SEQ ID NO: 5) (2 mg, final concentration: 1 mM) and the above deprotected Compound 8 (final concentration: 20 mM) were promptly dissolved in a 100 mM acetate buffer (pH 4), and the reaction was allowed to progress over 16 hours, while centrifugally concentrating. Thereafter, the reaction product was purified by HPLC to obtain 0.37 mg of Conjugate 13.

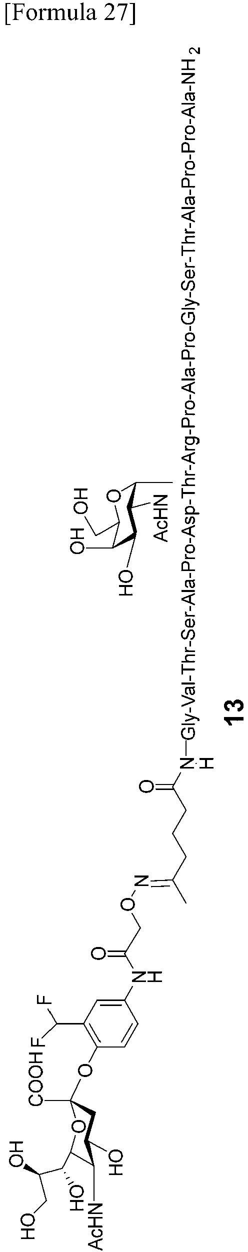

MALDI-TOF MS: m/z calcd for C₁₀₈H₁₆₈F₂N₂₇O₄₃ [M+H+]+ 2569.176, found 2569.137
(8-2) Incorporation of anti-MUC1 antibody uptake into lung cancer cells via binding to presented dynamic epitope.

A549 cells, which had been reported not to react with the anti-MUC1 antibody SN-131, were seeded on a glass plate for microscopic observation (20,000 cells/well, 200 µL of D-MEM medium containing 10% FBS) and were cultured for 24 hours at 37°C under 5% CO₂ conditions, so that the cells were allowed to adhere to the plate. Subsequently, (1) 20 µM Conjugate 13 (synthesized in (8-1) above), and (2) 20 µM Conjugate 13 (synthesized in (8-1) above) supplemented with 1 mM DANA (2,3-didehydro-2-deoxy-N-acetylneuraminic acid; inhibitory effect against NEU1: IC₅₀ = 49 ± 8 µM) were prepared, separately, and were each added to the cells after removing the medium. After the obtained mixture had been cultured for 2 hours at 37°C under 5% CO₂ conditions, the medium was removed, and the cells were then washed with the medium three times.

Thereafter, SN-131 (2 µg/mL, WO2011/135869) labeled with Hoechst 33342 (2.5 µg/mL) and Alexa488, and Opti-MEM medium were added, and the culture was continued for further 1 hour. After that, the resultant was washed twice with Opti-MEM, the cells were then observed under a fluorescence microscope (BIOREV BZ-9000, Keyence), and images acquired with a BZ-II analyzer were then analyzed.

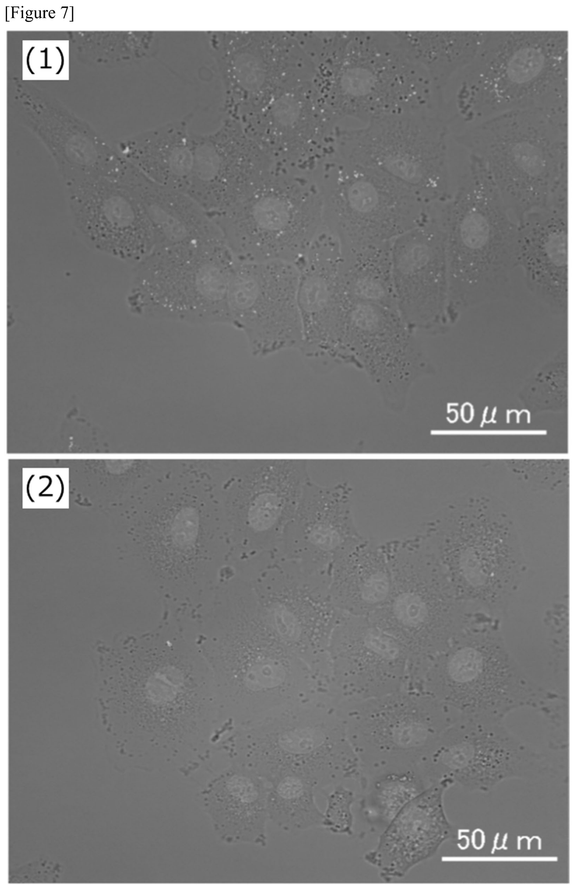

The results are shown in Figure 7. In this figure, after the reaction with Conjugate 13, the anti-MUC1 antibody SN-131 (green) added as shown in (1) was incorporated into the A549 cells (Figure 7(1)). However, this phenomenon was not observed when DANA, a low-molecular-weight reversible inhibitor against NEU1, was present in a high concentration, as shown in (2) (Figure 7(2)).

These results have demonstrated that the SN-131-MUC1-Tn-NEU1 conjugate was incorporated into the A549 cells via endocytosis through specific binding between the anti-MUC1 antibody SN-131 and MUC1-Tn (the dynamic epitope of SN-131) presented via a covalent bond to NEU1 on the surface of the cancer cell membrane, resulting from the irreversible reaction with Conjugate 13.

### Example 9: Synthesis of dynamic epitope

As an additional dynamic epitope, Conjugate 14 containing Antigen Glycopeptide 14 of fibronectin glycoprotein was synthesized. Conjugate 14 was synthesized according to the reaction scheme shown below.

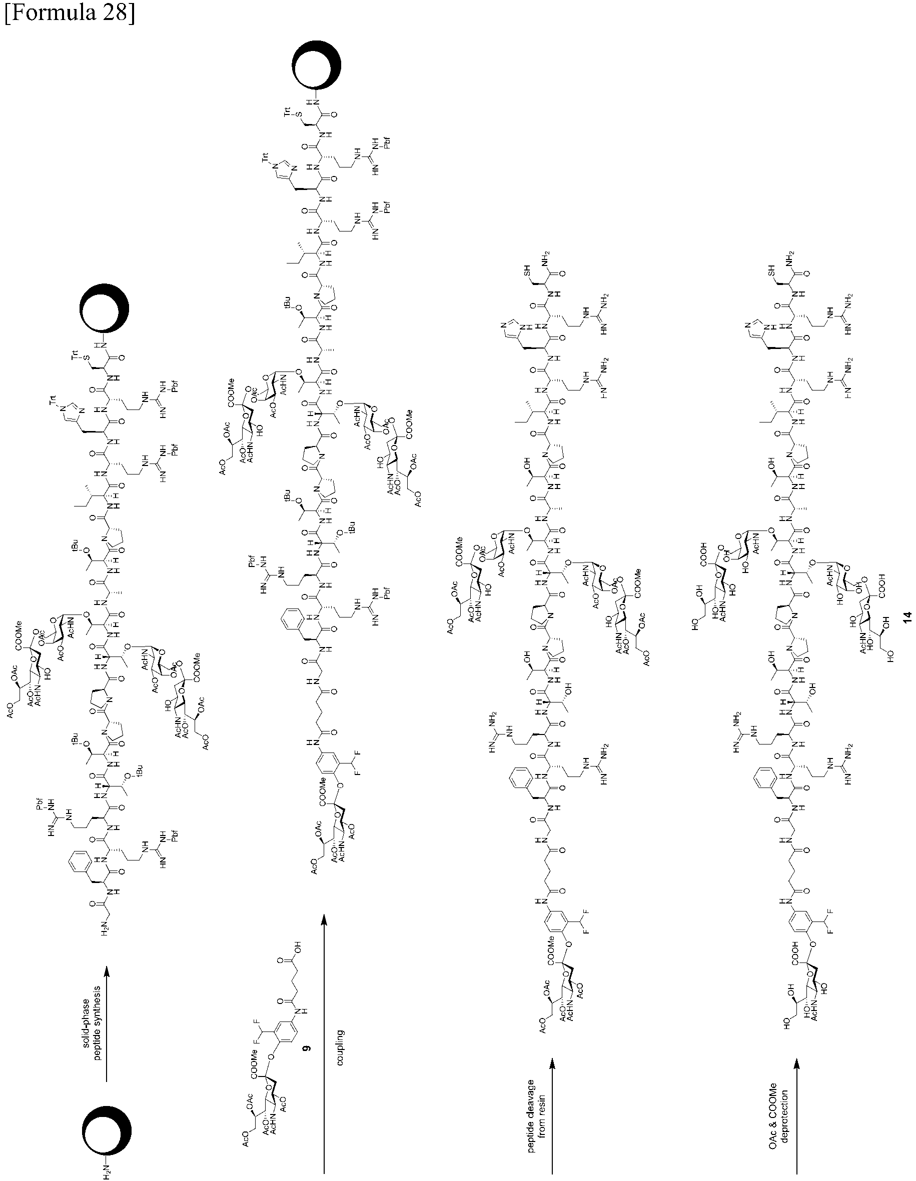

### Conjugate 14 (SEQ ID NO: 6): (NEU1 inhibitor)-²¹⁴⁷Gly-Phe-Arg-Arg-Thr-Thr-Pro-Pro-Thr(Siaα2,6GalNAcα1)-Thr(Siaα2,6GalNAcα1)-Ala-Thr-Pro-Ile-Arg-His-Arg²¹⁶³-Cys-NH₂

Glycopeptide Antigen 14 in the IIICS region of the fibronectin glycoprotein was synthesized by a Fmoc solid-phase peptide synthesis method. Basically, using Rink Amide-ChemMatrix resin (0.42 mmol/g, 100 mg, 21 µmol), individual steps were sequentially proceeded by performing a reaction for 10 minutes for each step, under microwave irradiation (50°C) in a DMF solution consisting of a Fmoc amino acid derivative (84 µmol), HBTU (84 µmol) and HOBt (84 µmol). Unreacted amino acids were acetylated by treating them using acetic anhydride/DIEA/DMF (v : v : v = 0.5 : 1 : 8.5) at room temperature for 30 seconds. The Fmoc groups were deprotected by treating them with 20% piperidine/DMF for 3 minutes under microwave irradiation (50°C).

The peptide of interest was synthesized by repeating the aforementioned three steps: (A) elongation of various types of Fmoc amino acids, (B) acetylation of unreacted amino groups, and (C) de-Fmocation. However, at the positions of Thr2155 and Thr2156, instead of the usual Fmoc amino acid derivative, an Fmoc glycoamino acid derivative, to which a disaccharide (Sialyl-Tn) consisting of sialic acid and N-acetylglucosamine bound, was separately produced by enU Pharma, Inc., and the synthetic reaction was proceeded using this Fmoc glycoamino acid derivative.

After condensation of all amino acids and glycoamino acids, Compound 9 (NEU1 Sialidase Inhibitor Derivative 9) synthesized in (1-2) of Example 1 was condensed to the N-terminal amino group under the same conditions as those for Fmoc amino acid elongation. Furthermore, trifluoroacetic acid : water : ethanedithiol : tetraisopropylsilane (v : v : v : v = 94 : 2.5 : 2.5 : 1) were added to the reaction system, and the obtained mixture was then shaken at room temperature for 2 hours, so that a synthetic intermediate of interest was cleaved from the solid-phase carrier, and at the same time, the side chain was deprotected.

The reaction solution was filtrated, tert-butylmethyl ether was then added, and the obtained mixture was then centrifuged (5000 G, 4°C, 10 minutes). The supernatant was discarded, and the precipitate was dissolved in a 50% acetonitrile aqueous solution, and was then freeze-dried to obtain a crude product of Conjugate 14 of interest. This freeze-dried product was dissolved in methanol : water (v : v = 2 : 1), and a 1 N sodium hydroxide aqueous solution was then added thereto to adjusted the pH to pH 12.5. The resulting solution was shaken at room temperature for 1 hour to deprotect acetyl groups in the glycan region. Thereafter, a 30% acetic acid aqueous solution was used to return the solution to neutral, and the solvent was then removed under reduced pressure conditions. The residue was purified by reversed-phase high performance liquid chromatography to obtain Conjugate 14 of interest.

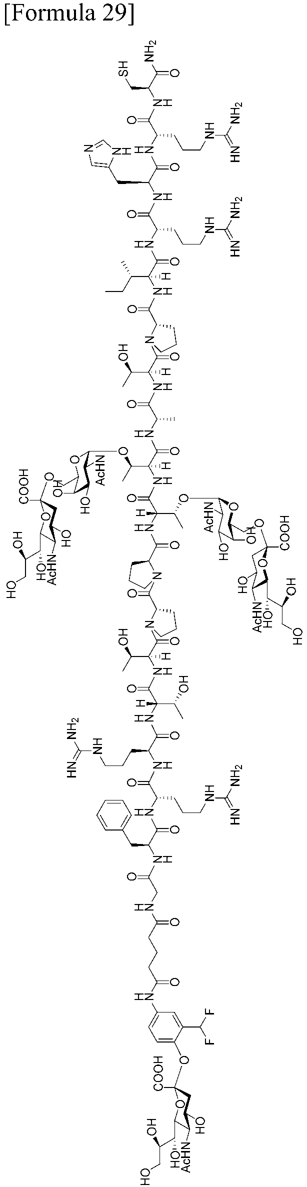

MALDI-TOFMS: m/z calcd for C149H235F2N39O60S [M-H]- 3599.615, found 3599.265.

Moreover, a conjugate having the following non-glycosylated peptide (SEQ ID NO: 7) used in the screening of antibodies was also synthesized in the same manner as described above.

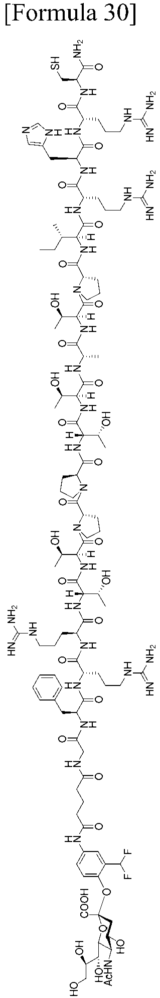

MALDI-TOF MS: m/z calcd for C111H175F2N35O34S [M-H]- 2611.265, found 2611.644.

### Example 10: Synthesis of dynamic epitope

Conjugate 14 produced in Example 9 was conjugated to KLH (carrier protein) according to a maleimidobenzoyloxysuccinimide (MBS) method to obtain a conjugate for use in antibody generation. Approximately 0.1 mg of the obtained conjugate for use in antibody generation was administered subcutaneously to the base of the tail of three mice (B6D2F1/Slc), so that an immune response was provoked. A booster immunization was administered, 17 days after the subcutaneous administration, by the same method as described above. Thereafter, blood was collected 4 days after, and iliac lymph nodes were then harvested. Lymphocytes collected from the harvested iliac lymph nodes were fused with myeloma cells SP2, and were then cultured in a HAT selection medium to select antibody-producing cells.

Next, the culture supernatant of the resulting hybridoma was dispensed on an ELISA plate. Positive wells were selected based on binding to a bovine serum albumin (BSA)-glycopeptide antigen conjugate and binding to a BSA-peptide antigen conjugate (a conjugate of BSA and the non-glycosylated peptide prepared in Example A). The positive wells were screened based on a binding reaction with the glycopeptide that was a dynamic epitope and the non-glycosylated peptide that was a control.

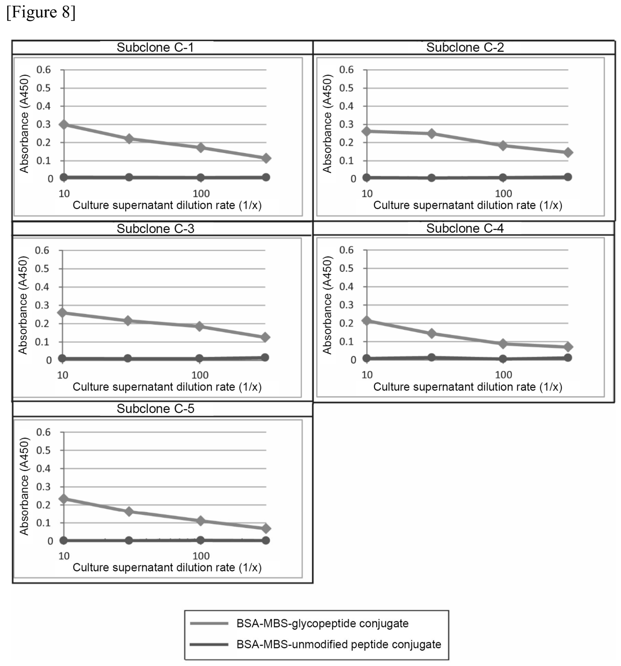

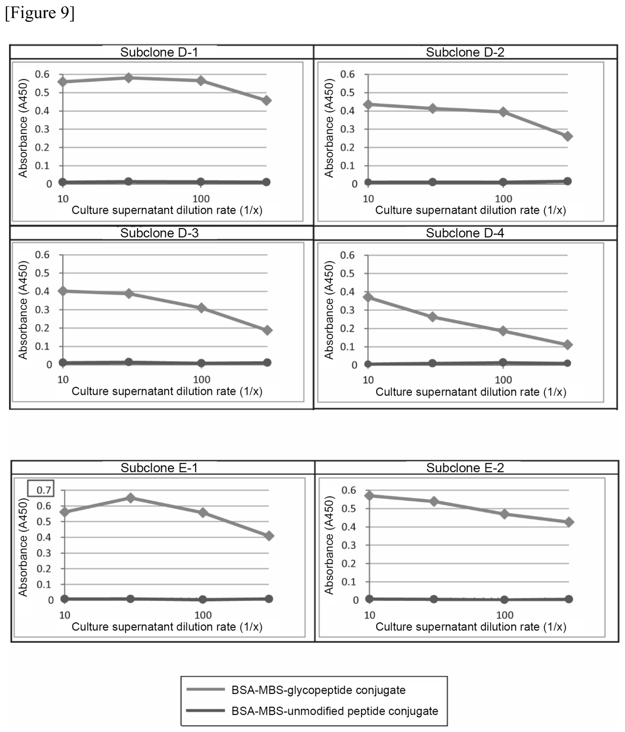

Fused cells were cloned by a limiting dilution method, etc. Figure 8 and Figure 9 show the binding specificity of the culture supernatants of single clones (subclone D-1 to subclone D-5, subclone E-1 to subclone E-4, and subclone F-1 to subclone F-2) obtained from three clone lines, clone D, clone E and clone F, to the glycopeptide (dynamic epitope). These results have demonstrated that the antibodies obtained from the culture supernatants of these clones bound only to the glycopeptide, and that these antibodies do not exhibit affinity for non-glycosylated peptide.

### Example 11: Synthesis of glycopeptides having different glycosylation patterns and evaluation of antigen recognition of antibodies

In order to examine the binding specificity of the monoclonal antibody against fibronectin glycoprotein produced in Example 10 to the fibronectin glycopeptide, a glycopeptide and a non-glycosylated peptide each having a different glycosylation pattern were synthesized, and the antigen recognition of the antibody in the culture supernatant was evaluated according to ELISA using a compound conjugated with BSA (or "substrate").

First, the following 6 types of glycopeptides for evaluation (SEQ ID NOs: 8 to 13) that target the three putative glycosylated sites, Thr2152, Thr2155 and Thr2156, of the fibronectin glycoprotein were synthesized according to the solid-phase synthesis protocols of Glycopeptide 14, using Fmoc-Thr[Ac3Sia *α* (2→6)Ac2GalNAc *α* 1→]-OH, Fmoc-Thr[Ac4Gal *β* (1→3)Ac2GalNAc *α* 1→]-OH, or Fmoc-Thr[Ac3GalNAc *α* 1→]-OH.

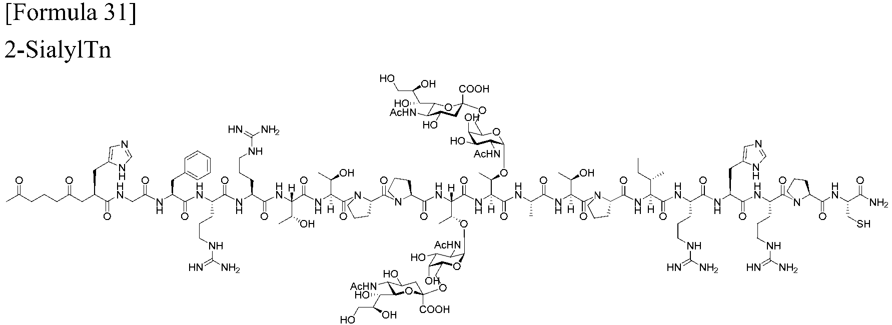

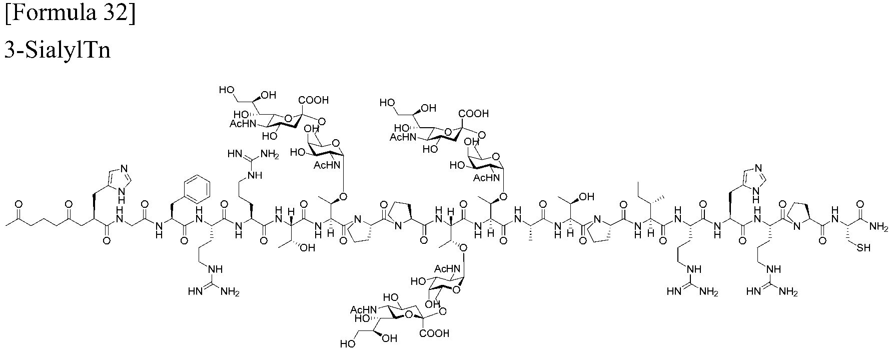

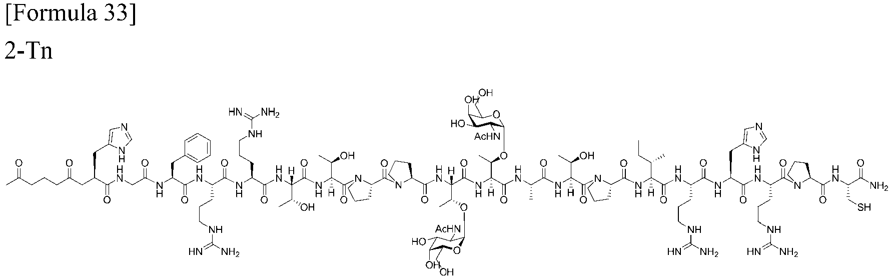

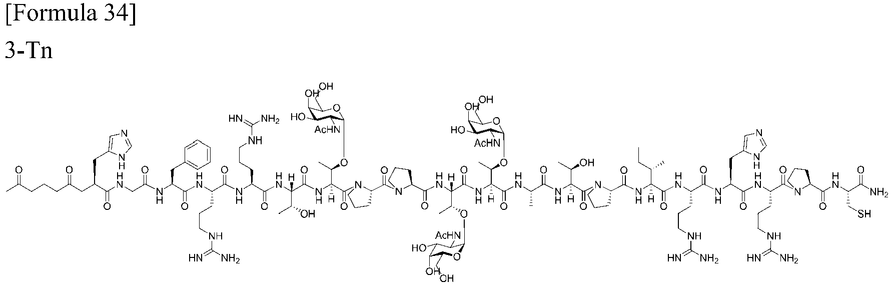

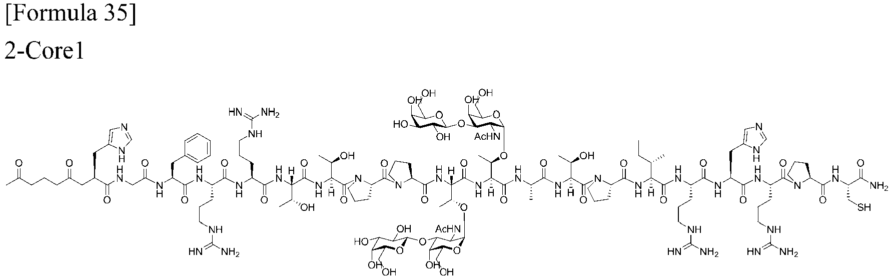

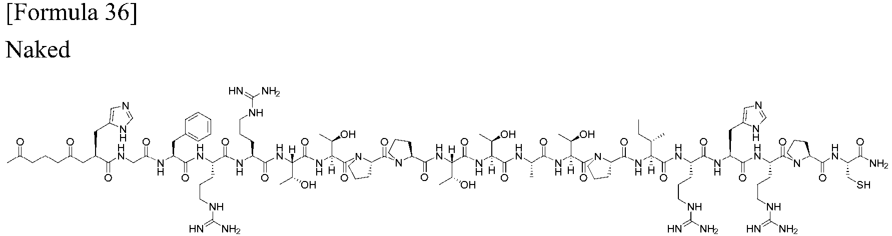

The synthesized glycopeptides were each conjugated with BSA to prepare BSA-glycopeptide conjugates. A PBS solution (50 µL), in which a BSA-peptide conjugate at a concentration of 1 µg/mL had been dissolved, was added to the wells of a nonspecific adsorprtion plate for ELISA (Sumitomo Bakelite Co., Ltd.), and was then left at rest at 4°C overnight. Thereafter, the solution was discarded, and the wells were washed with PBS. After that, 100 µL of PBS containing 1% BSA was added to the wells, and was then left at rest at room temperature for 2 hours to block the surface. After discarding the solution and washing the wells, each culture supernatant was diluted 10-fold, 30-fold, 100-fold, and 300-fold, with an ELISA buffer (25 mM Tris, 150 mM NaCl, 0.05% Tween-20, 0.1% BSA, pH 7.5), and 50 µL of each diluted culture supernatant was added to the plate, and was left at rest at room temperature for 1 hour. Thereafter, the wells were washed in the same manner as described above, and 50 µL of anti-mouse IgG antibody AP conjugate (Promega) that had been diluted to 1/2500 with the ELISA buffer was added, and was then left at rest at room temperature for 1 hour. The solution was discarded, and the wells were washed with PBS to completely remove the secondary antibody, and thereafter, a 4-nitrophenol phosphate solution was added. and the wells were incubated at room temperature for 30 minutes. The absorbance at 405 nm was measured using a plate reader.

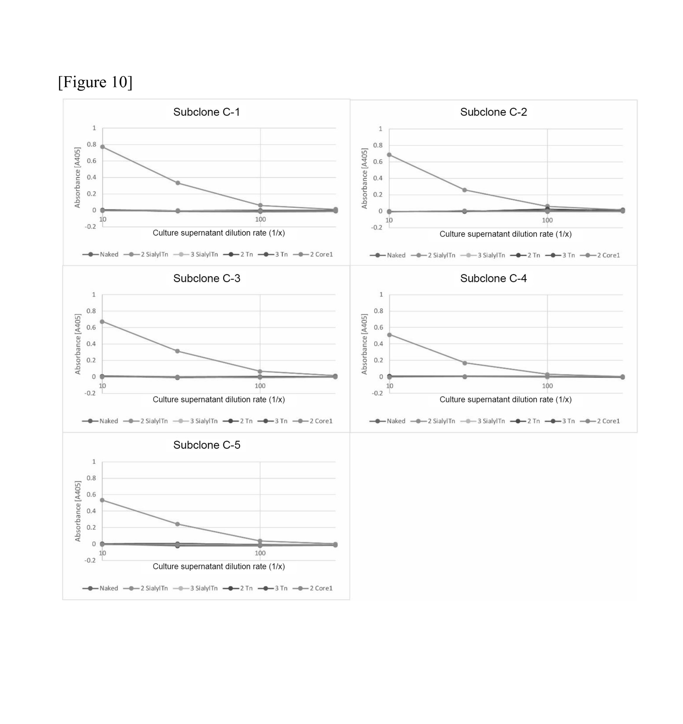

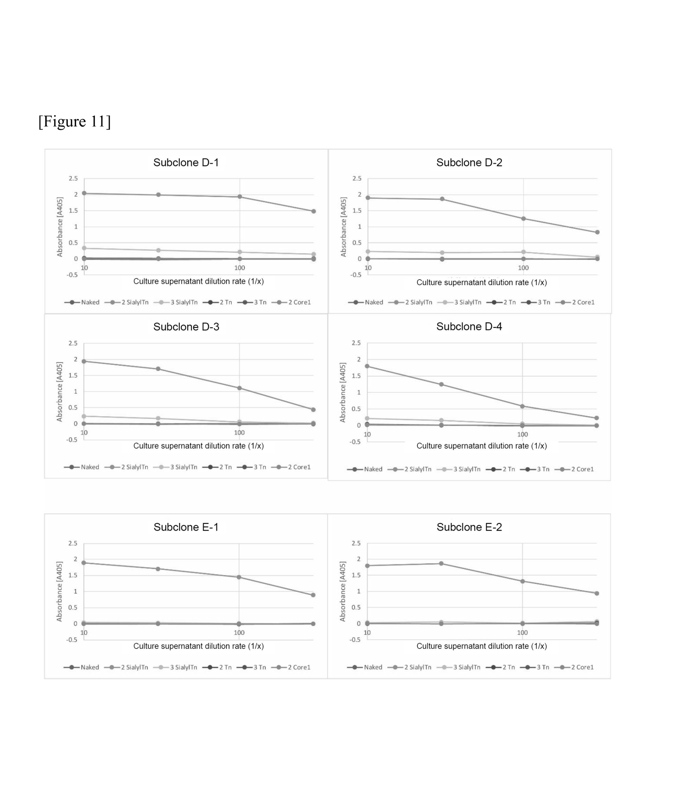

The results are shown in Figure 10 and Figure 11. It has been demonstrated that Clone C, Clone D, and Clone E all strongly bind to glycopeptides with Sialyl-Tn bound to two sites, Thr2155 and Thr2156, but that these clones do not bind to glycopeptides with a different glycosylation (Tn or T) at the same positions, or to glycopeptides with Sialyl-Tn bound to three sites, Thr2152, Thr2155 and Thr2156. It has been demonstrated that, by using the antigen presentation method of the present invention, highly antigen-specific antibodies that do not bind to glycopeptides with different glycosylation patterns are generated.
SEQ ID NO: 1: Glycopeptide Precursor 10 
Gln-Val-Ser-Pro-Phe-Gly-Glu-Val-Phe-Asn (Fucα1,6GlcNAcβ1)-Ala-Thr-Arg-Phe-Ala-
Ser-Val-Cys
SEQ ID NO: 2: Non-glycosylated peptide
   Gln-Val-Ser-Pro-Phe-Gly-Glu-Val-Phe-Asn-Ala-Thr-Arg-Phe-Ala-Ser-Val-Cys
SEQ ID NO: 3: Glycopeptide comprising two glycosylated sites Thr323 and Ser325 of SARS-CoV-2 spike glycoprotein 
Ser-Asn-Phe-Arg-Val-Gln-Pro-Thr (Galβ1,3GalNAcα1)-Glu-Ser (Galβ1,3GalNAcα1)-
Ile-Val-Arg-Phe-Asn-Ile-Cys
SEQ ID NO: 4: Non-glycosylated peptide of SARS-CoV-2 spike glycoprotein Ser-Asn-Phe-Arg-Val-Gln-Pro-Thr-Glu-Ser-Ile-Val-Arg-Phe-Asn-Ile-Cys
SEQ ID NO: 5: MUC1-Tn glycopeptide 
Gly-Val-Thr-Ser-Ala-Pro-Asp-Thr (GalNAcα1)-Arg-Pro-Ala-Pro-Gly-Ser-Thr-Ala-Pro-
Pro-Ala
SEQ ID NO: 6: Antigen glycopeptide of fibronectin glycoprotein 
Gly-Phe-Arg-Arg-Thr-Thr-Pro-Pro-Thr (Siaα2,6GalNAcα1)-Thr (Siaα2,6GalNAcα1)-
-Ala-Thr-Pro-Ile-Arg-His-Arg-Cys
SEQ ID NO: 7: Non-glycosylated peptide of antigen glycopeptide of fibronectin glycoprotein
   Gly-Phe-Arg-Arg-Thr-Thr-Pro-Pro-Thr-Thr-Ala-Thr-Pro-Ile-Arg-His-Arg-Cys
SEQ ID NO: 8: Glycopeptide (2-SialylTn) of Example 11 
His-Gly-Phe-Arg-Arg-Thr-Thr-Pro-Pro-Thr (Siaα2,6GalNAcα1)-Thr
(Siaα2,6GalNAcα1)-Ala-Thr-Pro-Ile-Arg-His-Arg-Pro-Cys
SEQ ID NO: 9: Glycopeptide (3-SialylTn) for evaluation, of Example 11 
His-Gly-Phe-Arg-Arg-Thr-Thr (Siaα2,6GalNAcα1)-Pro-Pro-Thr (Siaα2,6GalNAcα1)-
Thr (Siaα2,6GalNAcα1)-Ala-Thr-Pro-Ile-Arg-His-Arg-Pro-Cys
SEQ ID NO: 10: Glycopeptide (2-Tn) for evaluation, of Example 11 
His-Gly-Phe-Arg-Arg-Thr-Thr-Pro-Pro-Thr (GalNAcα1)-Thr (GalNAcα1)-Ala-Thr-Pro-
Ile-Arg-His-Arg-Pro-Cys
SEQ ID NO: 11: Glycopeptide (3-Tn) for evaluation, of Example 11 
His-Gly-Phe-Arg-Arg-Thr-Thr (GalNAcα1)-Pro-Pro-Thr (GalNAcα1)-Thr (GalNAcα1)-
-Ala-Thr-Pro-Ile-Arg-His-Arg-Pro-Cys
SEQ ID NO: 12: Glycopeptide (2-Core1) for evaluation, of Example 11 
His-Gly-Phe-Arg-Arg-Thr-Thr-Pro-Pro-Thr (Galβ1,3GalNAcα1)-Thr
(Galβ1,3GalNAcα1)-Ala-Thr-Pro-Ile-Arg-His-Arg-Pro-Cys
SEQ ID NO: 13: glycopeptide (Naked) for evaluation, of Example 11 His-Gly-Phe-Arg-Arg-Thr-Thr-Pro-Pro-Thr-Thr-Ala-Thr-Pro-Ile-Arg-His-Arg-Pro-Cys

### Industrial Applicability

According to the method of the present invention, an MHC-independent antigen presentation method has become possible. Thereby, since the glycan region of the "dynamic epitope" is effectively recognized by B cells (antigen-specific B cells are induced) without being decomposed, it has become possible to generate an antibody that specifically bind to the "dynamic epitope" of interest that is an antigen structure consisting of glycans and peptides, and thus, it has become possible to dramatically improve the efficiency of generating such antibodies. Furthermore, by presenting the dynamic epitope on the surface of, for example, cancer cells, it has been possible to establish a method for efficiently incorporating a cancer therapeutic antibody that binds to the dynamic epitope into the cancer cells.

## Claims

1. A conjugate represented by the following formula (1): 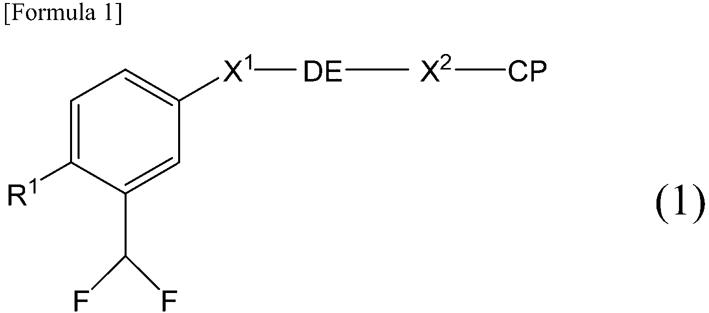 wherein R¹ represents a neuraminic acid group or a neuraminic acid derivative group, DE represents an antigenic site selected from a glycoprotein and a glycopeptide, CP represents a carrier site, and X¹ and X² each independently represent a single bond or a linking group.

2. The conjugate according to claim 1, wherein, in the above formula (1), R¹ is a group represented by the following formula (2): 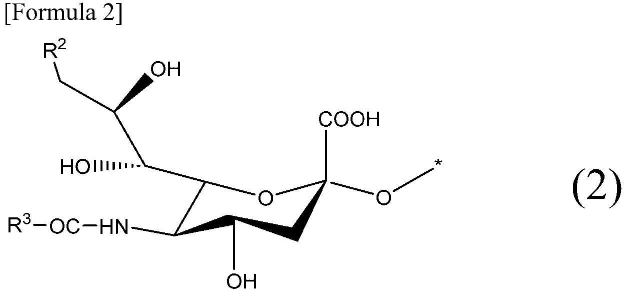 wherein R² represents a group containing at least one type selected from the group consisting of a carbon atom, an oxygen atom and a nitrogen atom, and also containing 10 or less carbon atoms, R³ represents a hydrocarbon group containing 1 to 12 carbon atoms, and * represents a binding site with a benzene ring in the formula (1).

3. The conjugate according to claim 2, wherein, in the above formula (2), R² represents a hydroxy group, an alkyl group containing 1 to 6 carbon atoms, or an alkylamide group containing 1 to 6 carbon atoms.

4. The conjugate according to claim 2 or 3, wherein, in the above formula (2), R³ represents an alkyl group containing 1 to 3 carbon atoms.

5. The conjugate according to claim 1 or 2, wherein the carrier site is at least one type selected from the group consisting of lipid nanoparticles, metal nanoparticles, polysaccharides, and carrier proteins.

6. The conjugate according to claim 1 or 2, which is used to bind to cells having Neuraminidase 1 (NEU1) or Neuraminidase 3 (NEU3).

7. The conjugate according to claim 1 or 2, which is for use in antibody generation.

8. A pharmaceutical composition, comprising the conjugate according to claim 1 or 2.

9. The pharmaceutical composition according to claim 8, which is used in prevention and/or treatment of a tumor and/or a cancer.

10. A vaccine, comprising the conjugate according to claim 1 or 2.

11. A method for obtaining antigen presenting cells, which present the antigenic site and the carrier site in the above formula (1) on the cell surface thereof,
the method comprising
allowing the conjugate according to claim 1 or 2 to come into contact with cells having Neuraminidase 1 (NEU1) or Neuraminidase 3 (NEU3).

12. A method for generating an antibody, comprising generating an antibody using the antigen presenting cells according to claim 11.

13. A method for treating a tumor and/or a cancer, comprising:
administering the conjugate according to claim 1 or 2, and
administering an antibody binding to the antigenic site in the above formula (1).

14. Use of the conjugate according to claim 1 or 2 for production of a preventive and/or therapeutic agent for a tumor and/or a cancer.

15. A conjugate represented by the following formula (11): 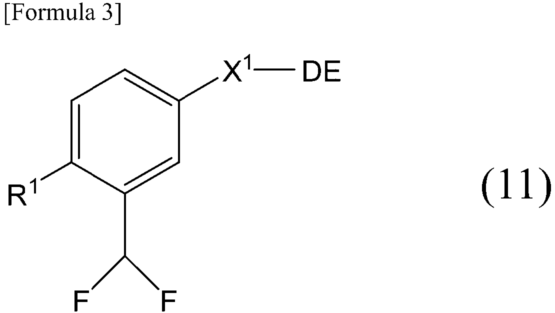 wherein R¹ represents a neuraminic acid group or a neuraminic acid derivative group, DE represents an antigenic site selected from a glycoprotein and a glycopeptide, and X¹ and X² each independently represent a single bond or a linking group.

16. The conjugate according to claim 15, wherein, in the above formula (11), R¹ is a group represented by the following formula (2): 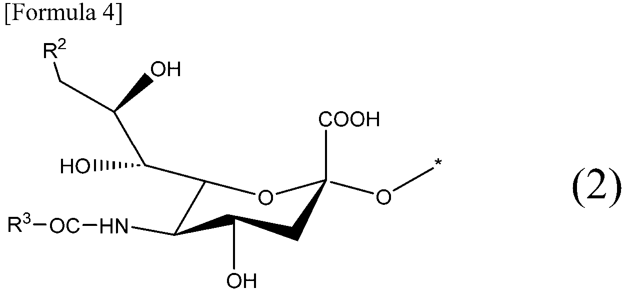 wherein R² represents a group containing at least one type selected from the group consisting of a carbon atom, an oxygen atom and a nitrogen atom, and also containing 10 or less carbon atoms, R³ represents a hydrocarbon group containing 1 to 12 carbon atoms, and * represents a binding site with a benzene ring in the formula (1).

17. The conjugate according to claim 16, wherein, in the above formula (2), R² represents a hydroxy group, an alkyl group containing 1 to 6 carbon atoms, or an alkylamide group containing 1 to 6 carbon atoms.

18. The conjugate according to claim 16 or 17, wherein, in the above formula (2), R³ represents an alkyl group containing 1 to 3 carbon atoms.

19. A pharmaceutical composition, comprising the conjugate according to claim 15 or 16.

20. A method for obtaining antigen presenting cells, which present the antigenic site and the carrier site in the above formula (11) on the cell surface thereof,
the method comprising
allowing the conjugate according to claim 15 or 16 to come into contact with cells having Neuraminidase 1 (NEU1) or Neuraminidase 3 (NEU3).
